(19) Europäisches Patentamt
European Patent Office
Office européen des brevets

(11) **EP 4 537 820 A1**

(12) **EUROPEAN PATENT APPLICATION**

(43) Date of publication:
**16.04.2025 Bulletin 2025/16**

(21) Application number: **23306787.5**

(22) Date of filing: **13.10.2023**

(51) International Patent Classification (IPC):
***A61K 31/17*** $^{(2006.01)}$ ***A61K 45/06*** $^{(2006.01)}$
***A61P 29/00*** $^{(2006.01)}$ ***A61P 31/00*** $^{(2006.01)}$
***A61P 31/12*** $^{(2006.01)}$ ***A61P 35/00*** $^{(2006.01)}$
***A61P 35/04*** $^{(2006.01)}$ ***A61P 37/04*** $^{(2006.01)}$

(52) Cooperative Patent Classification (CPC):
(C-Sets available)
**A61K 31/17; A61K 45/06; A61P 29/00; A61P 31/00; A61P 31/12; A61P 35/00; A61P 35/04; A61P 37/04** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Institut Gustave-Roussy**
  **94800 Villejuif (FR)**
- **Institut National de la Santé et de la Recherche Médicale**
  **75013 Paris (FR)**
- **Université Paris-Saclay**
  **91190 Gif-sur-Yvette (FR)**
- **Università degli Studi di Torino**
  **10124 Torino (IT)**

(72) Inventors:
- **APCHER, Sébastien**
  **95130 FRANCONVILLE (FR)**
- **LAULIN, Justine**
  **94800 VILLEJUIF (FR)**
- **CASCIO, Paolo**
  **11020 SAINT CHRISTOPHE AOSTA (IT)**

(74) Representative: **Cabinet Becker et Associés**
**25, rue Louis le Grand**
**75002 Paris (FR)**

Remarks:
The complete document including Reference Table(s) and the Sequence Listing(s) can be downloaded from the EPO website

# (54) COMPOUND AND COMBINATIONS COMPRISING SAID COMPOUND TO IMPROVE IMMUNE RESPONSE

(57) The present invention relates to the field of medicine. It more particularly relates to a compound of formula (I), and to pharmaceutical combinations and compositions comprising such a compound preferably together with at least one distinct therapeutic agent, as well as to uses thereof for treating an immunodeficient subject, in particular for preventing or treating a disease or symptom such as a cancer, an inflammation, a neurodegenerative disease or an infection.



Processed by Luminess, 75001 PARIS (FR)

(Cont. next page)

(52) Cooperative Patent Classification (CPC): (Cont.)

C-Sets
**A61K 31/17, A61K 2300/00**

## Description

The present invention relates to the field of medicine and is typically used in therapeutic and prophylactic areas. It more particularly relates to a compound of formula (I), and to pharmaceutical combinations and compositions comprising such a compound preferably together with at least one distinct therapeutic agent, as well as to uses thereof for treating an immunodeficient subject, in particular for preventing or treating a disease or symptom such as a cancer, an inflammation, a neurodegenerative disease or an infection.

## BACKGROUND OF THE INVENTION

The endogenous major histocompatibility class I (MHC-I) antigenic presentation pathway is a vital process for identifying and eliminating cells that pose a threat to the host such as cancer cells. While extensive research has focused on the cellular factors involved in this pathway, the origins of endogenous antigenic peptides remain poorly understood. Questions persist regarding the presence of multiple peptide sources, the significance of these sources for pathway functionality, and the cellular capacity to control the selection of peptide sources and its potential implications [Apcher, S. et al. (2023)]. Previous studies postulated that endogenous MHC-I restricted pathway peptides are not exclusively derived from the degradation of full-length proteins but can also arise from defective ribosomal products (DRiPs) and cryptic translation events [Shastri N. *et al.,* 2005; Yewdell J.W. *et al.,* 1996]. These events involve the synthesis of polypeptides through unconventional translational mechanisms, including intron sequences, intron/exon junctions, 5' and 3' untranslated regions, and alternate translational reading frames [Apcher S. *et al.,* 2022]. Such observations have redirected research towards alternative processes beyond protein degradation for antigenic production. Inventors' investigations have revealed that antigenic presentation remains comparable regardless of whether the peptides are expressed from intron or exon sequences. This is supported by the so-called Pioneer Translation Products (PTPs) which are generated through a translation event distinct from the canonical process that gives rise to full-length proteins [Apcher S. *et al.,* 2011; Apcher S. *et al.,* 2013; Sroka E.M. *et al.,* 2023]. Notably, blocking mRNA export from the nucleus to the cytoplasm significantly enhances the antigenic presentation of both exon and intron-encoded peptides. While inventors' previous work on PTPs was primarily focused on direct presentation and viral immune evasion mechanisms, inventors have recently demonstrated that PTPs can serve as a source of peptides for the exogenous MHC-I pathway, undergoing cross-presentation by professional antigen-presenting cells (pAPCs) to specifically activate naive CD8+ Tcells. PTPs have been discovered within exosomes, which are subsequently taken up by bone marrow dendritic cells, facilitating cross-presentation. Furthermore, they have reported that PTPs purified from tumors, referred to as tumor-associated PTPs (TA-PTPs), can be used in combination with exosomes to create a potent immune cancer vaccine [Duvallet E. *et al.,* 2016]. Irrespective of the peptide source, the production of MHC-I immune responses relies on the proteasomal system, the major proteolytic system in eukaryotic cells. At the core of this system lies the 20S proteasome or core particle, a multicatalytic protease complex regulated by several regulatory complexes [Vigneron N. *et al.,* 2014]. Among these regulatory complexes, the REG family has been shown to associate with the 20S proteasome, activating its three peptidase activities *in vitro* [Li, J. *et al.,* 2001]. The REG family, also known as 11S or PA28, consists of three distinct 28 kDa subunits (α, β, and γ), which have the capacity to assemble into two ring-shaped heptameric regulatory complexes, each weighing 200 kDa. These subunits are encoded by the PSME1, PSME2, and PSME3 genes, respectively. One of the complex, termed REGαβ, comprises three α and four β subunits and is predominantly found in the cytoplasm. The second regulatory complex, termed REGγ, consists exclusively of γ subunits and forms a homoheptameric structure primarily localized in the nucleus. These regulatory elements have the capability to bind to either or both ends of the 20S proteasome independently of ATP, leading to the opening of the 20S proteasome's gate [Cascio, P. *et al.*,2014]. While numerous studies have established a strong association between REGαβ and antigen production for the immune system [Rechsteiner M. *et al.,* 2000], the precise functions and mechanisms of action of REGγ remain elusive, as only a limited number of proteins whose degradation is mediated or regulated by REGγ have been described [Chen X, *et al.,* 2007; Li X. *et al.,* 2006; Li X. *et al.,* 2007]. Widely expressed in cells, REGγ is involved in cell growth, proliferation, apoptosis, chromatin organization, and DNA repair [Funderbirk K.E. *et al.,* 2022]. Furthermore, REGγ has emerged as a potential biomarker for cancer due to its high expression across various cancer types [Chen D. *et al.,* 2013; Kontos C.K. *et al.,* 2015; Song W. *et al.,* 2019] and potential therapeutic target [Chai F. *et al.,* 2014; Lei K. *et al.* ,2020] .

Initially, REGγ was documented to selectively augment the trypsin-like (T-L) activity of proteasomes [Realini C. *et al.,* 1997]. It has been reported that REGγ has the capability to allosterically enhance the T-L enzymatic activity of the β2 subunits of the 20S proteasome through long-range interactions [Thomas, T.A. *et al.,* 2022]. However, supplementary evidence has emerged, indicating that REGγ has the capacity to additionally stimulate the two other peptidase activities, namely chymotrypsin-like and caspase-like activities of the 20S core [Huang L. *et al.,* 2016; Jonik Nowak B. *et al.*,2018; Wilk S. *et al.,* 2000]. Surprisingly, REGγ can interact with and stimulate the activities of both standard and immunoproteasome 20S proteasomes. This interaction between REG family members and the proteasome has proven to be more intricate than previously believed. REG not only functions as an activator but also exerts an influence on the three-dimensional structure and dynamics of the proteasome [Thomas T.A. *et al.,* 2022; Lesne J. *et al.,* 2020].

The recent discovery of a connection between REGγ and antigenic peptide generation and presentation has provided novel insights into the complex mechanisms governing immune surveillance [Boulpicante M. *et al.,* 2020] revealing an inverse correlation between REGγ expression and the presentation of antigenic peptides on MHC-I molecules, observed both *in vitro* and *in vivo.* Further investigations have demonstrated that the binding of REGγ to the 20S catalytic core of the proteasome enhances its proteolytic activity. This specific activity has potential consequences, including the possibility of excessive degradation and subsequent destruction of antigenic peptides. Understanding the intricate interplay between REGγ, MHC-I complexes, formed by MHC-I molecules loaded with antigenic epitopes and proteolytic activity provides valuable insights into the regulation of antigenic peptide presentation and REGγ implications for immune responses.

Cancer is a critical global health-care problem with limited therapeutic options. Since cancers are lifethreatening illnesses, the identification of a promising oncotarget and its clinical correlates are relevant. Recently, inventors were able to develop a high-throughput screen where more than 2100 molecules from NIH compound libraries were tested to identify molecules that could inhibit the effect of REGγ on the 20S proteasome, either by blocking the interaction between the two complexes in view to inhibiting the negative role of REGγ on PTP-derived cancer immune responses.

Their findings shed light on the pivotal role of REGγ in shaping the immune landscape and provide valuable insights into the development of targeted therapies aimed at overcoming REGγ-mediated immunosuppression. Having unraveled the regulatory mechanisms of REGγ, inventors now herein provide novel treatment options that specifically target this regulator to enhance immune response, for example in the context of a cancer treatment but also in other pathological contexts such as neurodegeneration, infection, etc., and improve patient outcomes.

## BRIEF SUMMARY

Inventors herein describe a compound of formula (I):

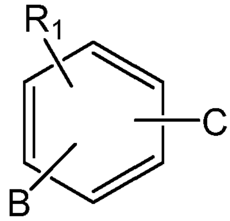

(I)

wherein

- $R_1$ is H or $C_1$-$C_6$ alkyl
- B is of formula (b):

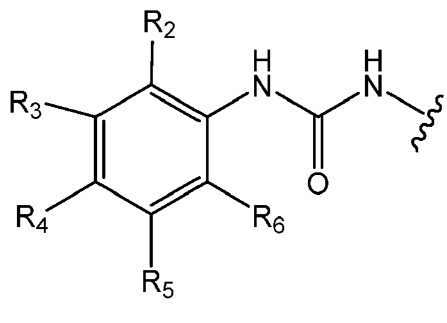

(b)

and

- C is of formula (c):

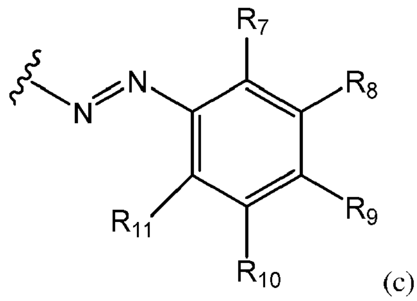

(c)

wherein:

each $R_2$, $R_3$, $R_4$, $R_5$, $R_6$, $R_7$, $R_8$, $R_9$, $R_{10}$, and $R_{11}$ is independently selected from H, halogen atom, -OH, $C_1$-$C_6$ alkyl, $C_1$-$C_6$ alkoxy, $C_1$-$C_6$ halogenoalkyl, 4- to 6-membered heterocycle preferably 4- to 6-membered heterocycloalkyl group, $C_1$-$C_6$ hydroxyalkyl, $-NO_2$, $-B(OR_{12})_2$, - $OP(O)(OR_{12})_2$, $-N(R_{12})_2$, $-CONHR_{12}$, $-SO_3R_{12}$ and $-COOR_{12}$ with $R_{12}$ is H or $C_1$-$C_6$ alkyl,

or a pharmaceutically acceptable salt and/or solvate thereof, in particular a compound of formula (Ia):

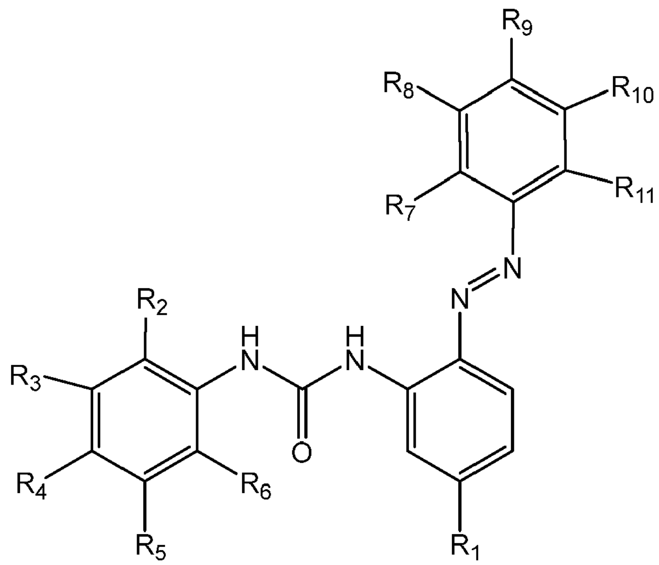

(Ia)

wherein $R_1$, $R_2$, $R_3$, $R_4$, $R_5$, $R_6$, $R_7$, $R_8$, $R_9$, $R_{10}$, and $R_{11}$ being as defined in claim 1, or a pharmaceutically acceptable salt and/or solvate thereof, for use for treating an immunodeficient subject.

Preferably, in the compound of formula (Ia):

- $R_1$ is H or $C_1$-$C_3$ alkyl, preferably $-CH_3$,
- $R_5$, $R_8$, $R_{10}$, and $R_{11}$ are H, and
- each $R_2$, $R_3$, $R_4$, $R_6$, $R_7$, and $R_9$, is independently selected from H, halogen atom, -OH, $C_1$-$C_3$ alkyl, $C_1$-$C_3$ alkoxy, $C_1$-$C_3$ halogenoalkyl, $C_1$-$C_3$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or $C_1$-$C_3$ alkyl, preferably $R_{12}$ is H.

In a preferred aspect, the compound of formula (Ia) is

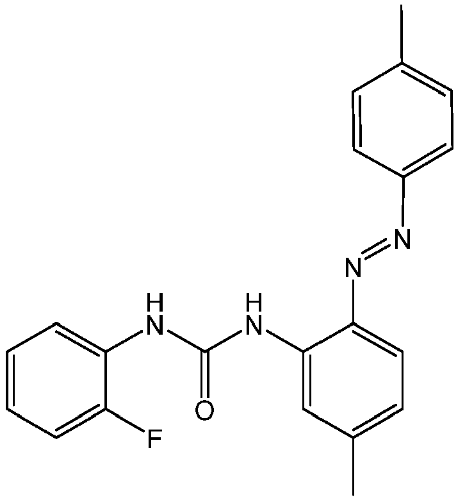

(compound 58)

or a pharmaceutically acceptable salt and/or solvate thereof.

Another object of the invention is a composition, preferably a pharmaceutical composition, in particular a vaccine, comprising a compound of the invention of formula (I), and a pharmaceutically acceptable carrier.

Another object of the invention is a combination of (a) a compound of the invention of formula (I) - also herein identified as "compound (a)" - and (b) a distinct therapeutic agent. The distinct therapeutic agent - also herein identified as "compound (b)" - is preferably selected from an immunotherapeutic agent, an anti-cancer agent and a combination thereof.

Also herein described is a composition comprising the combination of (a) and (b) and a pharmaceutically acceptable carrier.

Inventors herein provide compositions and combinations of the invention for use as a medicament.

They also provide a compound, composition, or combination of the invention, wherein said compound, combination or composition is for use for preventing or treating a disease, preferably a disease selected from a cancer, an inflammation, a neurodegenerative disease, and an infectious disease, preferably an infectious disease which is not induced by *M. tuberculosis,* in particular for use for treating a cancer in a subject, typically a cancer wherein, compared to the healthy cells of the subject, the tumor cells of the subject overexpress REGg.

Also herein described is a kit, in particular a pharmaceutical kit, for use in the prevention or treatment of a disease, preferably selected from a cancer, an inflammation, a neurodegenerative disease and an infectious disease, preferably an infectious disease which is not induced by *M. tuberculosis,* wherein the kit comprises i) a "compound (a)", in particular "compound 58", and

ii) an additional distinct therapeutic agent, in distinct containers.

## DETAILED DESCRIPTION

Unless defined otherwise, all technical and scientific terms used herein have the same meaning as commonly understood by one of ordinary skill in the art to which the subject-matter disclosed herein belongs.

## DEFINITIONS

The following definitions may be useful to understand embodiments as presented herein.

In the context of the present invention, the terms "immunodeficiency" and the adjectives "immunodeficient" and "immunocompromised" used to characterize a subject generally refer to a dysfunction of the immune system, in particular to a primary or acquired immunodeficiency. Immunodeficiency, also known as immunocompromisation, is a state in which the immune system's ability to fight infectious diseases and cancer is compromised or entirely absent. Most cases are acquired ("secondary") due to extrinsic factors that affect the patient's immune system.

In clinical settings, immunodepression by some drugs, such as steroids, can either be an adverse effect or the intended purpose of the treatment. Examples of such use is in organ transplant surgery as an antirejection measure and in patients with an overactive immune system, as in autoimmune diseases. A person who has an immunodeficiency of any kind is said to be immunocompromised. An immunocompromised individual may particularly be vulnerable to opportunistic infections, in addition to normal infections that could affect anyone. It also decreases cancer immunosurveillance, in which the immune system scans the body's cells and kills neoplastic ones. They are also more susceptible to infectious diseases owing to the reduced protection afforded by vaccines.

The distinction between primary versus secondary immunodeficiencies is based on, respectively, whether the cause originates in the immune system itself or is, in turn, due to insufficiency of a supporting component of it or an external decreasing factor of it.

Primary Immunodeficiency is also known as congenital immunodeficiencies. There are over 95 recognized primary immunodeficiency syndromes; they are generally grouped by the part of the immune system that is malfunctioning, such as lymphocytes or granulocytes.

Secondary immunodeficiencies, also known as acquired immunodeficiencies, can result from various immunosuppressive agents, for example, malnutrition, aging, particular medications (e.g., chemotherapy, disease-modifying antirheumatic drugs, immunosuppressive drugs after organ transplants, glucocorticoids) and environmental toxins like mercury and other heavy metals, pesticides and petrochemicals like styrene, dichlorobenzene, xylene, and ethylphenol. For medications, the term immunosuppression generally refers to both beneficial and potential adverse effects of decreasing the function of the immune system, while the term (iatrogenic) immunodeficiency generally refers solely to the adverse effect of increased risk for infection. Many specific diseases directly or indirectly cause immunosuppression. This includes many types of cancer, particularly those of the bone marrow and blood cells (leukemia, lymphoma, multiple myeloma), and certain chronic infections. Immunodeficiency is also the hallmark of acquired immunodeficiency syndrome (AIDS), caused by the human immunodeficiency virus (HIV). Various hormonal and metabolic disorders can also result in immune deficiency including anemia, hypothyroidism and hyperglycemia. Smoking, alcoholism and drug abuse also depress immune response.

Protein degradation is necessary for the body to maintain normal physiological function and homeostasis, but the disorder of degradation system may cause diseases [Lecker *et al.,* 2006]. In higher eukaryotes, between 80 and 90 % of proteins are degraded by the proteasome [T. Xie *et al.,* 2019]. The "proteasome system" or "proteasomal system" is involved in regulating cell cycle, cell death, signal pathway, and the immune response by degrading mutated or post-translationally damaged proteins [A. Ciechanover, 1998]. It was reported that 20S, forming complexes with PA700 (19S), PA28/REG (11S), and PA200, is the core component of the proteasome system. The 20S core is composed of four heptameric rings, including two $\alpha$ rings at both ends and two $\beta$ rings in the middle. It has been shown that $\alpha$ ring is responsible for binding activator and the $\beta$ ring contains active sites of many enzymes. Among them, the 26S proteasome system formed by 19S and 20S is ATP and ubiquitin-dependent, and is also the most widely studied proteasome system at present. In addition, the 20S can also degrade some abnormal proteins in an ATP- and ubiquitin-independent manner via combining with REG$\gamma$ which mediates the development of many diseases, including cancers [W. Baumeister *et al.,* 1998; Q. Zuo *et al.,* 2017].

The proteasome activator gamma ("REGgamma", "ReGg" or "REG$\gamma$"), also known as PA28$\gamma$, Ki antigen, and PSME3, was firstly identified in the serum of a systemic lupus erythematosus patient, belongs to a member of the 11S family of proteasome activators which also includes REG$\alpha$ and REG$\beta$ [L. Zhou *et al.,* 2019; T. Nikaido *et al.,* 1990]. Although approximately 50 % amino acid identity of REG$\alpha$ is the same as that of REG$\beta$, only about 25 % amino acid identity of REG$\alpha$ and REG$\beta$ can be observed in REG$\gamma$ [J. Li *et al.,* 2001]. REG$\alpha$ and REG$\beta$ are mainly located in the cytoplasm and were associated with the MHC-1-mediated immune response process in immune cells, while REG$\gamma$ is predominantly distributed in the nucleus and known to mediate the degradation of a series of proteins and the apoptosis of cells in an ATP- and ubiquitin-independent manner by binding specifically to and activating the 20S proteasome [X. Gao *et al.,* 2019; I. Mao *et al.,* 2008]. It is found that REG$\alpha$ and REG$\beta$ only exist in vertebrate bodies, while REG$\gamma$ can be observed in both vertebrates and invertebrates and is highly conserved [P. Masson *et al.,* 2001].

MHC class I molecules are one of two primary classes of major histocompatibility complex (MHC) molecules (the other being MHC class II) and are found on the cell surface of all nucleated cells in the bodies of vertebrates. They also occur on platelets, but not on red blood cells. The major histocompatibility complex (MHC) class I antigen presentation pathway allows the immune system to distinguish between self and non-self. The function of the MHC class I molecules is to display peptide fragments of proteins from within the cell to cytotoxic T cells (CTLs); this will trigger an immediate response from the immune system against a particular non-self-antigen displayed with the help of an MHC class I protein.

"MHC-I complex" are formed by MHC-I molecules loaded with antigenic epitopes.

Class I MHC molecules bind peptides generated mainly from degradation of cytosolic proteins by the proteasome. The MHC I:peptide complex is then inserted via endoplasmic reticulum into the external plasma membrane of the cell. The epitope peptide is bound on extracellular parts of the class I MHC molecule. Thus, the function of the class I MHC is to display intracellular proteins to (CTLs). However, class I MHC can also present peptides generated from exogenous proteins, in a process known as cross-presentation. A normal cell will display peptides from normal cellular protein turnover on its class I MHC, and CTLs will not be activated in response to them due to central and peripheral tolerance mechanisms. When a cell expresses foreign proteins, such as after viral infection, a fraction of the class I MHC will display these peptides on the cell surface. Consequently, CTLs specific for the MHC:peptide complex will recognize and kill presenting cells. Alternatively, class I MHC itself can serve as an inhibitory ligand for natural killer cells (NKs). Reduction in the normal levels of surface class I MHC, a mechanism employed by some viruses and certain tumors to evade CTL responses, activates NK cell killing.

"Peptides" are short chains of amino acids linked by peptide bonds. A particular peptide is a Pioneer Translation Product ("PTP"). PTPs are produced by a translation event distinct from the canonical event giving rise to full length proteins that takes place during the early scanning of newly synthesized mRNAs in the nuclear compartment (Apcher, Millot et al. 2013, Apcher, Daskalogianni et al. 2015). PTPs derive from non-spliced mRNA that are expressed from intron, exon, 3' and 5' untranslated regions (UTR), LncRNA (Long non coding RNA), miRNA (microRNA) and/or intergenic sequences. Preferably, the PTP consists in a sequence of 7 to 50 or 8 to 50 amino acid residues, for example of 7 to 27 amino acid residues, and have an atomic mass of 5 kDa or less, typically 3 kDa or less. A PTP can for example comprise 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46,47,48,49 or 50 amino acid residues. A PTP typically comprises a MHC class I epitope, and preferably also a MHC class II epitope.

A PTP can be obtained or purified from (and is said to be "derived from") any protein, polypeptide or antigen against which a specific immune response is to be elicited in the subject to be treated/vaccinated using standard biochemical approaches.

In the context of a tumor, PTP extraction involves the lysis of the tumor cell, in particular the lysis of its nucleus. A PTP purified from the nuclear compartment of a tumor cell [also identified as "tumor-associated PTP" (TA-PTPs)] typically elicits a specific anti-tumor $CD8^+$ T cell response against the tumor from which the tumor cell is derived. When present, the MHC class II epitope elicits a long lasting $CD4^+$ T cell response from the immune system which extends the anti-tumor CD8+ T cell response.

"Exosomes" are vesicles of endosomal origin that are secreted in the extracellular milieu following fusion of late endosomal multi vesicular bodies with the plasma membrane (Garin *et al.,* 2001; Thery *et al.,* 2002). Cells from various tissue types have been shown to secrete exosomes, such as dendritic cells, B lymphocytes, tumor cells and mast cells, for instance. Exosomes derived from tumor cells are herein identified as tumor-derived microvesicles. Exosomes from different origin exhibit discrete sets of proteins and lipid moieties (Thery *et al.,* 1999, Thery *et al.,* 2001). They notably contain proteins involved in antigen presentation and immuno-modulation indicating that exosomes play a role in cell-cell communications leading to the modulation of immune responses. Indeed, exosomes from dendritic cells (DC) pulsed with peptides derived from tumor antigens elicit anti-tumor responses in animal model using the matching tumor (Wolfers *et al.,* 2001, Zitvogel *et al.,* 1998).

Methods of producing, purifying or using exosomes for therapeutic purposes or as research tools have been described for instance in WO99/03499, WO00/44389 and WO97/05900, incorporated therein by reference. Recombinant exosomes have been described in the art, which derived from cells transfected with plasmids encoding recombinant proteins. Such recombinant exosomes contain the plasmidencoded recombinant protein (WO00/28001). Methods of manipulating the protein content of exosomes and of displaying antigens, adjuvant and markers for therapeutic purposes or as research tools have been described in WO03/016522.

Particular exosomes comprise/express PTPs, either naturally such as exosomes derived from a tumor cell, or purposively if the exosome is a recombinant exosomes genetically modified to express PTPs.

The term "subject" or "individual" refers to an animal, typically a mammal. Examples of mammals include humans and non-human animals such as, without limitation, domesticated animals (e.g., cows, sheep, cats, dogs, and horses), non-human primates (such as monkeys), rabbits, and rodents (e.g., mice and rats).The treatment is preferably intended for a human being in need thereof, whatever its age or sex. In the context of the present invention, a preferred subject is an immunodeficient subject, i.e., a subject suffering of a dysfunction of the immune system.

A particular subject suffers of acquired immunodeficiency for example immunodeficiency induced by a disease such as a cancer or an infection, by undernutrition or malnutrition, by irradiation, or by a medical treatment (iatrogenic immunodeficiency) such as for example a cancer treatment, a corticosteroid therapy, a transplant, etc..

Another particular subject, who may also be immunodeficient, suffers from a cancer, an inflammation, a neurodegenerative disease and/or an infection, or is considered "at risk of developing" such a disease or symptom, in which this has to be prevented.

The cancer patient may have a tumor. Unless otherwise specified in the present disclosure, the tumor is a cancerous or malignant tumor.

In some embodiments of each of the methods, combinations and uses herein, the combination therapy is administered to a subject who is previously untreated, i.e. is treatment naive-subject.

In some preferred embodiments of each of the methods, combinations and uses herein, the combination therapy is administered to a subject who has failed to achieve a sustained response after at least one prior therapy, i.e. is a treatment experienced-subject.

In a particular aspect in which the subject is a human being, that subject may be from various biogeographic origins. The subject may have a European phenotype, a Middle Eastern phenotype, a North African phenotype, a Horn of Africa phenotype, an Asian phenotype, or a phenotype specific to southern central Asia or northern South Asia. In a particular aspect, the human being is an Asian subject, for example a Chinese or Japanese subject.

Unless otherwise indicated, the terms "cancer", "cancerous tumor", "malignant tumor", "tumor", "neoplasia",

"cancer disease", or "proliferative disease", are herein used interchangeably. These terms refer to or describe the physiological condition in a mammal that is typically characterized by unregulated cell growth. As used herein, "cancer" may be any kind of cancer or neoplasia. This term refers to any malignant and/or invasive growth or tumor caused by abnormal cell growth.

The term "cancer" refers in particular to solid tumors named from the type of cells that form them, in particular of epithelial, neuroectodermal or mesenchymal origin, or refers to a liquid cancer such as a cancer of blood, bone marrow, or the lymphatic system. Examples of solid tumors include but are not limited to sarcomas and carcinomas. Examples of cancers of the blood include but are not limited to leukemias, lymphomas and myeloma. The term "cancer" includes but is not limited to a primary cancer that originates at a specific site in the body. The term cancer also includes a cancer that has metastasized, i.e., that has spread from the place in which it started to other parts of the body, for example to the central nervous system (CNS) in particular to the brain, to the bone, to a lung, or to the liver; a recurrence from the original primary cancer after remission, and a second primary cancer that is a new primary cancer in a person with a history of previous cancer of a different type from latter one. The secondary invasions (metastasis), for example from melanoma, lung cancer, kidney cancer, breast cancer, and colon cancer, may be loco-regional or distant metastases.

In a particular aspect, the cancer is associated to dysregulated REGγ. In a particular aspect, the cancerous tumor or tumor cells overexpress "REGy". A particular cancer of interest in the context of the invention is a cancer wherein, compared to the healthy cells of a subject, the tumor cells of said subject overexpress REGg.

Such a cancer associated to overexpression of REGγ may be for example a head and neck cancer, in particular head and neck squamous cell carcinoma, a thyroid carcinoma, a esophageal carcinoma, a breast cancer in particular a breast invasive carcinoma, a lung cancer in particular a non-small-cell lung cancer, a lung adenocarcinoma or a lung squamous cell carcinoma, a liver hepatocellular carcinoma, a cholangiocarcinoma, a renal cancer in particular a kidney renal clear cell carcinoma, a kidney chromophobe or kidney renal papillary cell carcinoma, a bladder urothelial carcinoma, a prostate cancer in particular a prostate adenocarcinoma, a skin cancer in particular a skin cutaneous melanoma, a rectum adenocarcinoma, a uterine corpus endometrial carcinoma, a colon or colorectal cancer in particular a colon adenocarcinoma, a gastric cancer in particular a stomach adenocarcinoma, an oral squamous cell carcinoma, a multiple myeloma, and a pancreatic cancer. The cancer is for example a metastatic cancer or a cancer resistant and/or refractory to standard-of-care treatment, in particular to immunotherapy.

An "infection" or "infectious disease" is the invasion of tissues by pathogens, their multiplication, and the reaction of host tissues to the infectious agent and the toxins they produce.

An infection is not synonymous with an infectious disease, as some infections do not cause illness in a host. In a preferred aspect, the subject considered in the context of the present invention is suffering of an infectious disease, also known as a transmissible disease or communicable disease, i.e., an illness resulting from an infection. The subject may suffer from a primary or secondary infection. While a primary infection can practically be viewed as the root cause of an individual's current health problem, a secondary infection is a sequela or complication of that root cause. For example, an infection due to a burn or penetrating trauma (the root cause) is a secondary infection. Primary pathogens often cause primary infection and often cause secondary infection. Usually, opportunistic infections are viewed as secondary infections (because immunodeficiency or injury was the predisposing factor). Other types of infection consist of mixed, iatrogenic, nosocomial, and community-acquired infection.

Infections can be caused by a wide range of pathogens, most prominently bacteria ("bacterial infection") such as *Staphylococcus aureus, Escherichia coli, Clostridium botulinum* and *Salmonella spp.,* and viruses and related agents such as viroids ("viral infection"). In a particular and preferred aspect, the infection is not associated or due to *Mycobacterium tuberculosis.*

Other pathogens include fungi, parasites, arthropods such as ticks, mites, fleas and lice, and prions. Hosts can fight infections using their immune systems. Mammalian hosts react to infections with an innate response, often involving inflammation, followed by an adaptive response.

Infectious diseases resulted in 9.2 million deaths in 2013 (about 17% of all deaths).

"Inflammation" or "inflammatory diseases" involve a pathogenic inflammatory response in the anatomical structure. The term "inflammation" designates both a disease or a symptom of a disease. In a preferred aspect, the subject considered in the context of the present invention is suffering of an inflammation or inflammatory disease resulting from immunodeficiency. In a particular aspect, the inflammation or inflammatory disease is induced by a cancer or an infection affecting or having affected the subject or is involved in neurodegeneration in the subject.

Considering cancer more particularly, inflammation orchestrates the microenvironment around tumor(s), contributing to proliferation, survival and migration. Cancer cells use selectins, chemokines and their receptors for invasion, migration and metastasis. According to a review of 2009, recent data suggests that cancer-related inflammation (CRI) may lead to accumulation of random genetic alterations in cancer cells. At present, chronic inflammation is estimated to contribute to approximately 15% to 25% of human cancers.

An "autoimmune disease" is a condition that results from an anomalous response of the adaptive immune system, wherein it mistakenly targets and attacks healthy, functioning parts of the body as if they were foreign organisms.

A "neurodegenerative disease" is caused by the progressive loss of structure or function of neurons, in the process known as neurodegeneration. Such neuronal damage may ultimately involve cell death. Neurodegenerative diseases include Amyotrophic lateral sclerosis ("ALS"), multiple sclerosis, Parkinson's disease, Alzheimer's disease, Huntington's disease, Batten disease, multiple system atrophy, and prion diseases such as Creutzfeld-Jakob's disease. Neurodegeneration can be found in the brain at many different levels of neuronal circuitry, ranging from molecular to systemic. Because there is no known way to reverse the progressive degeneration of neurons, these diseases are considered to be incurable; however research has shown that the two major contributing factors to neurodegeneration are oxidative stress and inflammation. Biomedical research has revealed many similarities between these diseases at the subcellular level, including atypical protein assemblies (like proteinopathy) and induced cell death. These similarities suggest that therapeutic advances against one neurodegenerative disease might ameliorate other diseases as well. Within neurodegenerative diseases, it is estimated that 55 million people worldwide had dementia in 2019, and that by 2050 this figure will increase to 139 million people.

As used herein, the term "treatment" or "treat" refers to therapeutic intervention in an attempt to alter the natural course of the subject being treated, and can be performed either for preventive (prophylactic) or curative purpose. Desirable effects of treatment include, but are not limited to, preventing occurrence or recurrence of disease, alleviation of symptoms, and diminishment of any direct or indirect pathological consequences of the disease, decreasing the rate of disease progression, amelioration or palliation of the disease state, and remission or improved prognosis. In preferred embodiments, products, combinations, compositions and methods of the invention are used to delay development of a disease, for example of a cancer, or to slow the progression of a disease, typically of tumor growth.

The treatment will induce a therapeutic response of the immune system of the subject, typically CD4+ and/or CD8+ T cells response(s). By inducing a T cell response is meant herein that a T cell response directed towards a certain antigen is elicited. Before said induction, said T cell response was not present, or below detection levels or not functional. By enhancing a T cell response is meant herein that the overall action of T cells directed towards a certain antigen is made higher and/or more efficient compared to the overall action of said T cells before said enhancement. For instance, after said enhancement more T cells directed towards said antigen may be generated. As a result, the action of the additionally generated T cells increases the overall action against said antigen. Alternatively, said enhancement may comprise the increment of the action of T cells directed towards said antigen. Said T cells may for instance react stronger and/or quicker with said antigen. Of course, the result of said enhancement may be generation of additional T cells together with increment of the action of said T cells. Alternatively, said enhancement may comprise generation of additional T cells, or increment of the action of T cells, only.

As used herein, by "a therapeutically effective amount" is meant an amount of the compound of the invention which prevents, removes, slows down the disease or disorder or reduces or delays one or several symptoms of said disease in the subject.

The herein mentioned compound of formula (I) is also herein identified as "compound (a)", and the herein mentioned "distinct therapeutic agent" is not a compound of formula (I) and is also herein identified as "compound (b)".

As used herein, an "Alkyl" refers to a saturated hydrocarbon radical which can be a straight or branched chain or a cyclic hydrocarbon group. For instance, a $C_1$-$C_6$ alkyl encompasses, without being limited to, methyl, ethyl, propyl, isopropyl, cyclopropyl, butyl, isobutyl, tert-butyl, cyclobutyl, pentyl, cyclopentyl, hexyl, and cyclohexyl. A $C_1$-$C_3$ alkyl encompasses for instance methyl, ethyl, propyl, isopropyl, and cyclopropyl.

"Halogen" denotes a halogen atom such as Cl, Br, I, or F. Preferred halogens are Cl and F.

An "alkoxy" refers to a radical of formula R-O- wherein R represents an alkyl group. Examples of alkoxy (or $C_1$-$C_6$ alkoxy) include for instance, methoxy, ethoxy, propoxy, isopropoxy, butoxy, pentoxy, hexyloxy.

A "hydroxyalkyl" refers to a radical of formula -A-OH wherein A represents an alkylene group.

A "halogenoalkyl" refers to a alkylene group substituted with one or several halogen atoms. Examples of halogenoalkyl are $-CF_3$ or $-CH_2$-$CHF_2$.

An "Alkylene group" refers to a bivalent alkyl group.

As used herein, the term "heterocycle" refers to a saturated or unsaturated, aliphatic or aromatic cyclic hydrocarbon group in which at least one ring carbon atom has been replaced with a heteroatom, preferably selected from nitrogen, oxygen, or sulfur atom. Advantageously, the heterocycle comprises from 3 to 10 ring atoms, preferably 3, 4, 5 or 6 ring atoms, wherein at least one of the ring atoms is a heteroatom such as nitrogen, oxygen or sulphur atom. The term "heterocycle" encompasses heteroaryl groups as well as aliphatic heterocyclic groups. For instance, examples of heterocycles encompass cycloheteroalkyl groups, namely a cycloalkyl wherein one or several carbon atoms have been replaced by a heteroatom. The term "heterocycle" includes for instance aziridinyl, azepanyl, diazepanyl, dioxolanyl, benzo [1,3] dioxolyl, azetidinyl, oxetanyl, pyrazolinyl, pyranyl, thiomorpholinyl, pyrazolidinyl, piperidyl, piperazinyl, 1,4-dioxanyl, imidazolinyl, pyridyl, pyrrolinyl, pyrrolidinyl, piperidinyl, imidazolidinyl, morpholinyl, 1,4-dithianyl, pyrrolidinyl, pyrimidinyl, oxozolinyl, oxazolidinyl, isoxazolinyl, isoxazolidinyl, thiooxetanyl, thiopyranyl, thiomorpholinyl, thiazolinyl, thiazolidinyl, isothiazolinyl, isothiazolidinyl, dihydropyranyl, dihydrofuranyl, dihydrothiopyranyl, pyrrolyl, thienyl, quinolyl, furanyl, isoquinolyl, dihydrothiophene, dihydropiperidinyl, tetrahydropiperidinyl, tetrahydrothiopyranyl, tetrahydropyranyl, tetrahydrofuranyl, and tetrahydrothiophene. In some embodiments, the "heterocycle" is a "heterocycloalkyl group" comprising 3 to 6 carbon atoms and one or two heteroatoms as ring atoms.

Heterocycloalkyl groups include piperidinyl, pyrrolidinyl, piperazinyl, thiomorpholinyl and morpholinyl.

The term "substituted" means that one or more hydrogen atoms on the designated atom or group are replaced by a non-hydrogen substituent, provided that the designated atom's normal valency under the existing circumstances is not exceeded. The terms "optionally substituted" can be replaced with terms "substituted or unsubstituted" throughout this application.

As used herein, the term "one or more" or "at least one" means 1 or more than 1, e.g. 1, 2, 3, 4 or 5, particularly 1, 2, 3 or 4, more particularly 1, 2 or 3.

As used herein, the term "pharmaceutically acceptable" refers to compositions, compounds, salts or solvates and the like that are, within the scope of sound medical judgment, suitable for contact with the tissues of the subject, or which can be administered to the subject, without excessive toxicity or other complications commensurate with a reasonable benefit/risk ratio.

The term "about" which is used to modify a numerically defined parameter means that the parameter may vary by as much as 10% above or below the stated numerical value for that parameter. For example, a dose of "about 5 mg" means 5 mg ± 10%, i.e., the dose may vary between 4.5 mg and 5.5 mg.

PRODUCT **("compound (a)"), and derivatives**

The Invention relates to a compound of formula (I):

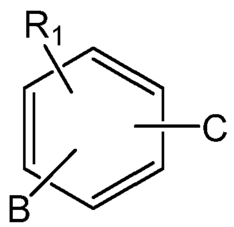

(I)

wherein

- $R_1$ is H or $C_1$-$C_6$ alkyl
- B is of formula (b):

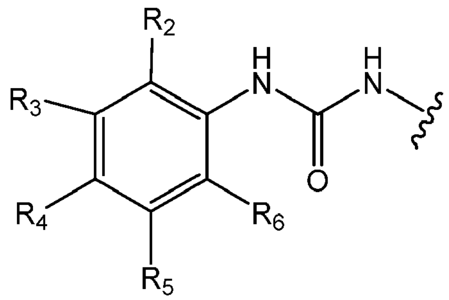

(b)

and

- C is of formula (c):

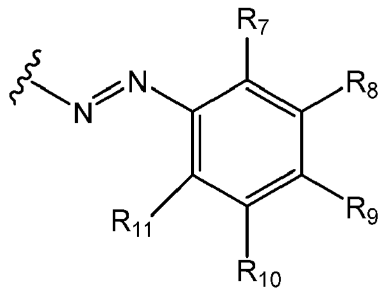

(c)

wherein:

each $R_2$, $R_3$, $R_4$, $R_5$, $R_6$, $R_7$, $R_8$, $R_9$, $R_{10}$, and $R_{11}$ is independently selected from H, halogen atom, -OH, $C_1$-$C_6$ alkyl, $C_1$-$C_6$ alkoxy, $C_1$-$C_6$ halogenoalkyl, 4- to 6-membered heterocycles preferably 4- to 6-membered heterocycloalkyl group, $C_1$-$C_6$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or $C_1$-$C_6$ alkyl, or a pharmaceutically acceptable salt and/or solvate thereof.

B, C and $R_1$ can be at any position on the phenyl ring. For instance, B can be at position meta, para, or ortho with respect to C. In a particular embodiment, B is at position ortho or para with respect to C.

In some embodiments, $R_1$ is H or a $C_1$-$C_3$ alkyl, preferably H or a methyl.

In a particular embodiment, the compound of formula (I) is such that at least three groups among $R_2$, $R_3$, $R_4$, $R_5$ and $R_6$ are H.

In some other embodiments, the compounds of formula (I) is such that at least three groups $R_7$, $R_8$, $R_9$, $R_{10}$ and $R_{11}$ are H.

In another embodiment, the compound of formula (I) is such that: - $R_1$ is H or $C_1$-$C_3$ alkyl, preferably H or $-CH_3$ and - each $R_2$, $R_3$, $R_4$, $R_5$, $R_6$, $R_7$, $R_8$, $R_9$, $R_{10}$, and $R_{11}$ is independently selected from H, halogen atom, -OH, $C_1$-$C_6$ alkyl, $C_1$-$C_6$ alkoxy, $C_1$-$C_6$ halogenoalkyl, $C_1$-$C_6$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or $C_1$-$C_6$ alkyl.

In a particular, embodiment, the compound of formula (I) is such that:

- $R_1$ is H or $C_1$-$C_3$ alkyl, preferably H or $-CH_3$ and
- each $R_2$, $R_3$, $R_4$, $R_5$, $R_6$, $R_7$, $R_8$, $R_9$, $R_{10}$, and $R_{11}$ is independently selected from H, halogen atom, -OH, $C_1$-$C_3$ alkyl, $C_1$-$C_3$ alkoxy, $C_1$-$C_3$ halogenoalkyl, $C_1$-$C_3$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or $C_1$-$C_3$ alkyl.

In another embodiment, the compound of formula (I) is such that:

- $R_1$ is H or $C_1$-$C_3$ alkyl, preferably H or $-CH_3$ and
- each $R_2$, $R_3$, $R_4$, $R_5$, $R_6$, $R_7$, $R_8$, $R_9$, $R_{10}$, and $R_{11}$ is independently selected from H, halogen atom preferably -Cl, -Br or -F, $C_1$-$C_3$ alkyl, OH, $C_1$-$C_3$ alkoxy, $C_1$-$C_3$ halogenoalkyl such as $CF_3$, -COOH, $-OPO(OH)_2$, $B(OH)_2$, $-SO_3H$, $-NH_2$ and $-NO_2$.

In the above embodiments, at least three groups among $R_2$, $R_3$, $R_4$, $R_5$ and $R_6$ may be H, and at least three groups among $R_7$, $R_8$, $R_9$, $R_{10}$ and $R_{11}$ may be H.

For illustration only, the compound of formula (I) may be such that each $R_2$, $R_3$, $R_4$, $R_5$, $R_6$, $R_7$, $R_8$, $R_9$, $R_{10}$, and $R_{11}$ is independently selected from H, a halogen atom preferably -Cl, -Br or -F, $C_1$-$C_3$ alkyl such as methyl, $C_1$-$C_3$ halogenoalkyl such as $CF_3$, and $-NO_2$ with proviso that at least three groups among $R_2$, $R_3$, $R_4$, $R_5$ and $R_6$ are H, and at least three groups among $R_7$, $R_8$, $R_9$, $R_{10}$ and $R_{11}$ are H.

As a further example, the compound of formula (I) may be such that each $R_2$, $R_3$, $R_4$, $R_5$, $R_6$, $R_7$, $R_8$, $R_9$, $R_{10}$, and $R_{11}$ is independently selected from H, halogen atom, preferably -F or -Cl, $C_1$-$C_6$ alkyl preferably $C_1$-$C_3$ alkyl, and $-NO_2$.

In some embodiments, the compound of formula (I) is such that $R_2$, $R_3$, $R_4$, $R_5$, $R_6$, $R_7$, $R_8$, $R_9$, $R_{10}$, and $R_{11}$ are not selected from halogenoalkyl groups.

In a particular embodiment, the compound of formula (I) is such that B is on position ortho with respect to C. In such an embodiment, R1 may be at position meta with respect to B (and thus at position para with respect to C).

Accordingly, in a further aspect, the Invention relates to a compound of formula (Ia):

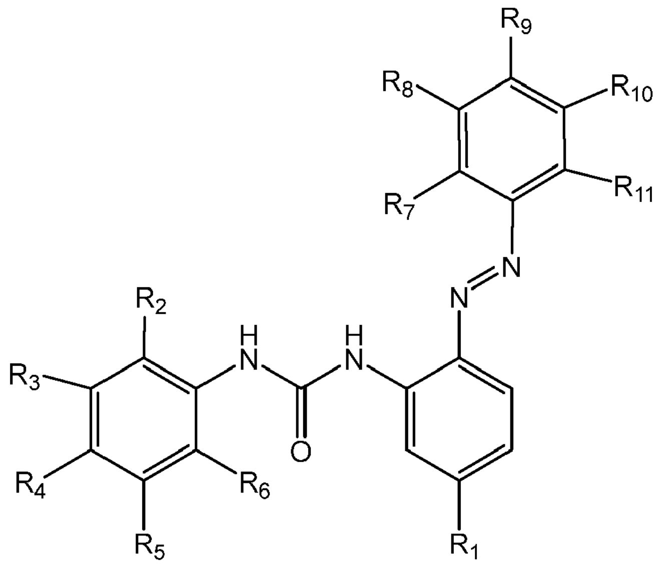

(Ia)

wherein $R_1$, $R_2$, $R_3$, $R_4$, $R_5$, $R_6$, $R_7$, $R_8$, $R_9$, $R_{10}$, and $R_{11}$ being as defined in formula (I) hereabove, or a pharmaceutically acceptable salt and/or solvate thereof.

In a particular embodiment, $R_1$ is H or $C_1$-$C_3$ alkyl. $R_1$ is preferably methyl.

In other embodiments, each $R_2$, $R_3$, $R_4$, $R_5$, $R_6$, $R_7$, $R_8$, $R_9$, $R_{10}$, and $R_{11}$ is independently selected from H, halogen atom, -OH, $C_1$-$C_6$ alkyl, $C_1$-$C_6$ alkoxy, $C_1$-$C_6$ halogenoalkyl, $C_1$-$C_6$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or $C_1$-$C_3$ alkyl, preferably $R_{12}$ is H.

In a particular embodiment, each $R_2$, $R_3$, $R_4$, $R_5$, $R_6$, $R_7$, $R_8$, $R_9$, $R_{10}$, and $R_{11}$ is independently selected from H, halogen atom preferably -Cl, -Br or -F, $C_1$-$C_3$ alkyl, OH, $C_1$-$C_3$ alkoxy, $C_1$-$C_3$ halogenoalkyl such as $CF_3$, -COOH, $-OPO(OH)_2$, $B(OH)_2$, $-SO_3H$, $-NH_2$ and $-NO_2$.

In a more particular embodiment, the compound of formula (Ia) is such that:

- R, is H or $C_1$-$C_3$ alkyl, preferably $-CH_3$,
- $R_5$, $R_8$, $R_{10}$, and $R_{11}$ are H, and
- each $R_2$, $R_3$, $R_4$, $R_6$, $R_7$, and $R_9$, is independently selected from H, halogen atom, -OH, $C_1$-$C_3$ alkyl, $C_1$-$C_3$ alkoxy, $C_1$-$C_3$ halogenoalkyl, $C_1$-$C_3$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or $C_1$-$C_3$ alkyl, preferably $R_{12}$ is H.

In a particular embodiment, the compound is of formula (Ib) or a pharmaceutically salt and/or solvate thereof:

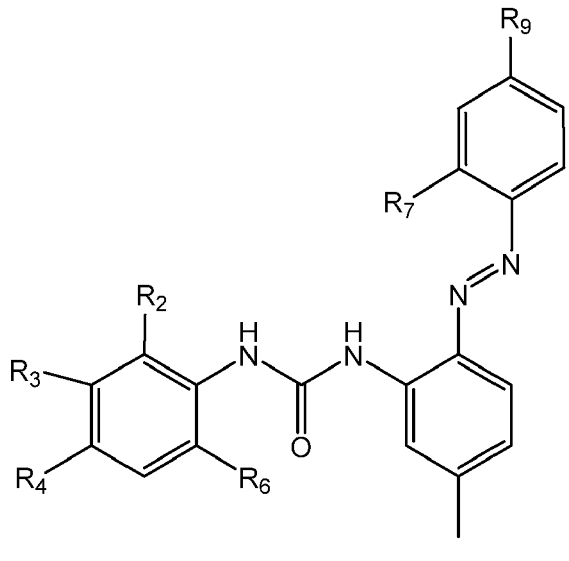

(Ib)

wherein $R_2$, $R_3$, $R_4$, $R_6$, $R_7$, and $R_9$ are as defined above for formula (I) or (Ia).

In some embodiments, the compound of formula (Ib) is such that each $R_2$, $R_3$, $R_4$, $R_6$, $R_7$, and $R_9$ is independently selected from the group consisting of H, halogen atom, -OH, $C_1$-$C_6$ alkyl, $C_1$-$C_6$ alkoxy, $C_1$-$C_6$ halogenoalkyl, $C_1$-$C_3$ hydroxyalkyl, -NO2, -B($OR_{12}$)$_2$, -OP(O)($OR_{12}$)$_2$, -N($R_{12}$)$_2$, -CONH$R_{12}$, - $SO_3R_{12}$ and -COO$R_{12}$ with $R_{12}$ is H or $C_1$-$C_3$ alkyl, preferably $R_{12}$ is H.

For instance, the compound of the Invention is of formula (Ib), wherein:

- each $R_2$, $R_3$, $R_4$, $R_6$, $R_7$, and $R_9$ is selected from H, halogen, such as -Cl, -F, $C_1$-$C_3$ alkyl such as methyl, $C_1$-$C_3$ halogenoalkyl such as $CF_3$, and -$NO_2$, and
- $R_5$, $R_8$, $R_{10}$, and $R_{11}$ are H.

In some embodiments, at least one group among $R_2$, $R_3$, and $R_4$ is H and/or at least one group among $R_7$, and $R_9$ is H.

In an additional aspect, the compound of the Invention is of formula (Ic) :

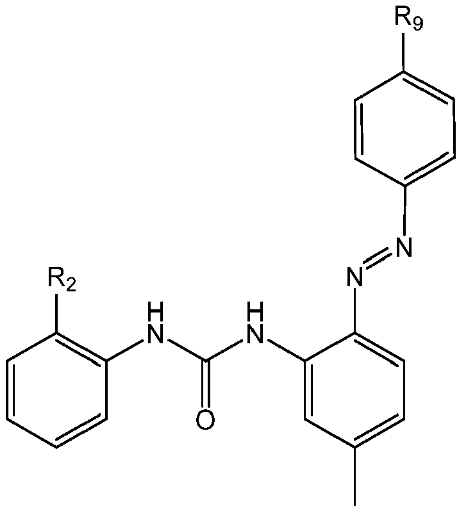

(Ic)

wherein $R_9$ and $R_2$ are as defined above in any one of Formula (I), (Ia) and (Ib), or a pharmaceutically acceptable salt or solvate thereof.

For instance, $R_2$ is a halogen atom and $R_9$ is $C_1$-$C_3$ alkyl.

In a preferred embodiment, the compound of the Invention is

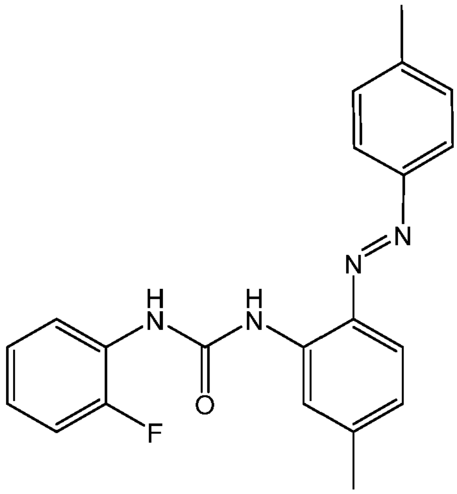

(compound 58)

or a pharmaceutically acceptable salt and/or solvate thereof.

Accordingly, in a further aspect, the Invention relates to a compound of formula (I) as defined above wherein B is at position para with respect to C. $R_1$ can be at position meta or ortho with respect to B, in particular in position ortho.

In particular, such a compound can be of formula (Id)

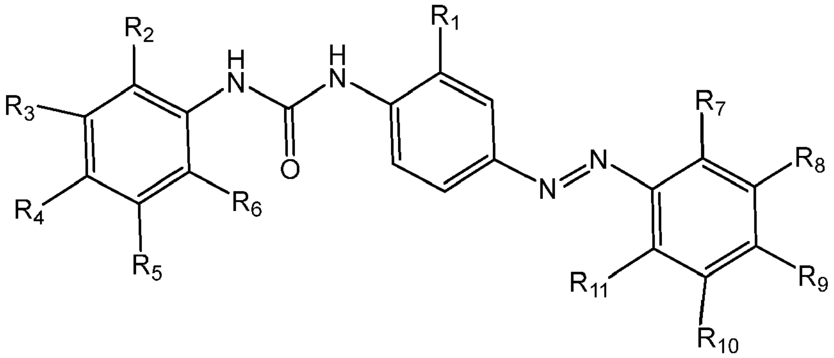

(Id)

wherein $R_1$, $R_2$, $R_3$, $R_4$, $R_5$, $R_6$, $R_7$, $R_8$, $R_9$, $R_{10}$, and $R_{11}$ being as defined in formula (I) hereabove, or a pharmaceutically acceptable salt and/or solvate thereof.

In a particular embodiment, $R_1$ is H or a $C_1$-$C_3$ alkyl. $R_1$ is preferably H or methyl.

In other embodiments, each $R_2$, $R_3$, $R_4$, $R_5$, $R_6$, $R_7$, $R_8$, $R_9$, $R_{10}$, and $R_{11}$ is independently selected from H, halogen atom, -OH, $C_1$-$C_6$ alkyl, $C_1$-$C_6$ alkoxy, $C_1$-$C_6$ halogenoalkyl, a $C_1$-$C_6$ hydroxyalkyl, $-NO_2$, $-B(OR_{12})_2$, -OP(O)$(OR_{12})_2$, $-N(R_{12})_2$, $-CONHR_{12}$, $-SO_3R_{12}$ and $-COOR_{12}$ with $R_{12}$ is H or $C_1$-$C_3$ alkyl, preferably $R_{12}$ is H.

In a particular embodiment, each $R_2$, $R_3$, $R_4$, $R_5$, $R_6$, $R_7$, $R_8$, $R_9$, $R_{10}$, and $R_{11}$ is independently selected from H, halogen atom preferably -Cl, -Br or -F, $C_1$-$C_3$ alkyl, OH, $C_1$-$C_3$ alkoxy, $C_1$-$C_3$ halogenoalkyl such as $CF_3$, -COOH, $-OPO(OH)_2$, $B(OH)_2$, $-SO_3H$, $-NH_2$ and $-NO_2$.

In a more particular embodiment, the compound of formula (Id) is such that:

- $R_1$ is H or $-CH_3$,
- each $R_2$, $R_3$, $R_4$, $R_6$, $R_8$, $R_9$ is independently selected from H, halogen atom, -OH, $C_1$-$C_3$ alkyl, $C_1$-$C_3$ alkoxy, $C_1$-$C_3$ halogenoalkyl, $C_1$-$C_3$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or $C_1$-$C_3$ alkyl, preferably $R_{12}$ is H, and
- $R_1$, $R_5$, $R_7$, $R_{10}$ and $R_{11}$ are H.

As mentioned above, a preferred compound of the invention is

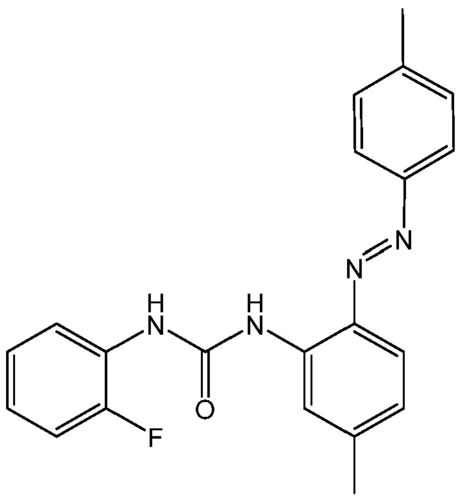

Other possible compounds of interest are shown in Figure 1B.

The compounds of the Invention can be prepared by standard chemical synthesis as described in the prior art. For instance, the synthesis of the compound can comprise a step of a preparing an aromatic azobenzene derivatives bearing an halide by oxidation of aromatic amine in the presence of an appropriate catalyst, and a step of reacting of said aromatic azobenzene derivatives bearing an halide with a phenylurea derivative, e.g. via copper or palladium-catalyzed amidation.

Certain compounds of the invention are also commercially available.

It goes without saying that the present invention includes all possible stereoisomers of the compounds of the invention as described above, in particular of formula (I), (Ia)-(Id) and compound 58.

Certain compounds of the invention can comprise one or several chiral centers. Thus, the present invention also includes all the diastereo-isomers and all enantiomers, e.g. (R) or (S) of the compounds of the invention in isolated forms as well as any mixtures thereof in any ratio, e.g. in racemic mixtures for enantiomers. Isolation of a single stereoisomer of a compound of the invention, e.g. a single enantiomer or a single diastereoisomer, of said compound can be achieved by any suitable method described in the state in the art, such as recrystallization or chromatography, especially chiral chromatography.

The invention also includes all possible tautomers of the compounds of the invention and any mixture of said tautomers in any ratio.

The present invention also encompasses isotopic forms of the compounds of the invention in particular those in which one or more hydrogen atom(s) are replaced, for example, by deuterium.

The present invention further encompasses conjugates of a compound as described herein, in particular of formula (I) and (Ia)-(Id), with a polar and/or hydrophilic group preferably selected from polyols in particular saccharides or oligosaccharides, polyethers such as polyethylene glycols, amino acid residues or oligopeptides.

The present invention also encompasses all possible polymorphic or pseudo-polymorphic forms of the compounds of the invention. The compounds of the invention may be in amorphous state or in crystalline state.

The compounds of the present invention can exist as a hydrate or as a solvate, which means that the compounds of the present invention can contain solvent molecules, in particular molecules of water or alcohol such as isopropanol or ethanol, as structural element of its crystal lattice. Thus, the present invention includes all hydrates or solvates of the compounds of the invention.

In preferred embodiments, the compound of the invention is in anhydrous form or is an hydrate.

The compounds of the present invention may exist in free form, e.g. as a free base, or as a free acid, or as a zwitterion, or in the form of a salt. Depending on the functional group present in the compounds, acid-addition salt or base-addition salt can be prepared.

Typically, the compound of the invention may be in any salt, either an organic or inorganic addition salt, particularly any pharmaceutically acceptable organic or inorganic addition salt, or which is used, for example, for isolating or purifying the compounds of the present invention.

In some embodiments, the compound of the invention is in the form of an acid-addition salt.

Acid-addition salt can be formed by reaction with an inorganic acid, or "mineral acid", such as hydrochloric, hydrobromic, hydroiodic, sulfuric, sulfamic, bisulfuric, phosphoric, or nitric acid, for example, or with an organic acid, such as formic, acetic, pyruvic, trifluoroacetic, propionic, butyric, hexanoic, heptanoic, citric, tartaric, stearic, lactic, oxalic, malonic, succinic, malic, adipic, this list being not exhaustive.

In some other embodiments, the compound of the invention is in the form of a base-addition salt.

Bases that can be used to obtain such salts are for instance alkali metal hydroxides, including potassium hydroxide, sodium hydroxide and lithium hydroxide; alkaline earth metal hydroxides, such as barium hydroxide and calcium hydroxide; and organic bases, such as piperidine, diethanolamine and N-methylglucamine, this list being not exhaustive.

Said salt forms of the compounds of the invention can be prepared by methods well known by the skilled artisan, e.g., by contacting the compounds of the invention in free state with an appropriate base or acid.

## COMPOSITIONS AND COMBINATIONS

Inventors herein describe combinations and compositions, more particularly pharmaceutical combinations and compositions.

A "pharmaceutical composition" refers to a mixture of one or more of the therapeutic agents described herein, or a pharmaceutically acceptable polymorph, enantiomer, stereoisomer, salt, solvate, tautomer, hydrate or prodrug thereof as an active ingredient, and at least one pharmaceutically acceptable carrier or excipient. In some embodiments, the pharmaceutical composition comprises two or more pharmaceutically acceptable carriers and/or excipients.

Thus, "pharmaceutical composition" typically means one or more active ingredients, and one or more inert ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients.

The (pharmaceutical) combinations or pharmaceutical compositions of the invention include combinations and compositions suitable for oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the condition(s) being treated and on the nature of the active ingredient(s). They may be conveniently presented in unit dosage form and prepared by any of the methods well-known in the art of pharmacy.

The pharmaceutical composition or combination of the invention is typically a vaccine. Indeed, the main goal of

vaccination is to induce an effective immune response that can prevent or treat a disease (or a symptom thereof) as herein described in the "definition" section. Such a vaccine can for example control an infectious disease or a cancer in a subject in need thereof.

In a preferred aspect, the combination or composition comprises, in addition to the compound of formula (I) ("compound (a)"), preferably in addition to the compound identified as "compound 58", at least one distinct therapeutic agent ("compound (b)"), and possibly a pharmaceutically acceptable carrier.

As used herein, a "pharmaceutically acceptable carrier" refers to a carrier, excipient or diluent that does not cause significant irritation to an organism and does not abrogate the biological activity and properties of the active compound or therapeutic agent. The pharmaceutical acceptable carrier may comprise any conventional pharmaceutical carrier or excipient. The choice of carrier and/or excipient will to a large extent depend on factors such as the particular mode of administration, the effect of the excipient on solubility and stability, and the nature of the dosage form. Suitable pharmaceutical carriers include inert diluents or fillers, water, and various organic solvents (such as hydrates and solvates). The pharmaceutical compositions may, if desired, contain additional ingredients such as flavorings, binders, excipients, and the like. Thus, for oral administration, tablets containing various excipients, such as citric acid may be employed together with various disintegrants such as starch, alginic acid and certain complex silicates and with binding agents such as sucrose, gelatin and acacia. Examples, without limitation, of excipients include calcium carbonate, calcium phosphate, various sugars and types of starch, cellulose derivatives, gelatin, vegetable oils and polyethylene glycols. Additionally, lubricating agents such as magnesium stearate, sodium lauryl sulfate and talc are often useful for tableting purposes. Solid compositions of a similar type may also be employed in soft and hard filled gelatin capsules. Non-limiting examples of materials, therefore, include lactose or milk sugar and high molecular weight polyethylene glycols. When aqueous suspensions or elixirs are desired for oral administration the active compound therein may be combined with various sweetening or flavoring agents, coloring matters or dyes and, if desired, emulsifying agents or suspending agents, together with diluents such as water, ethanol, propylene glycol, glycerin, or combinations thereof.

The at least one distinct therapeutic agent ("compound(b)") may be for example an immunotherapeutic agent, an anti-cancer agent, a therapeutic agent used to treat an infection, an inflammation or inflammatory disease, and any combination thereof. In particular aspect, the "compound(b)" may be a medication used to prevent or treat a neurodegenerative disease. In another particular aspect, it may be PTPs and/or microvesicles such as exosomes comprising PTPs.

The least one distinct therapeutic agent herein identified as "compound(b)" is preferably an immunotherapeutic agent and/or an anti-cancer agent.

***Immunotherapeutic agents* / *Immunotherapy***

Immunotherapeutic agents refer to therapeutic agents used in the context of immunotherapy, and reference to immunotherapy refer to the use of immunotherapeutic agents.

Examples of immunotherapeutic agents includes monoclonal antibodies, granulocyte colonystimulating factor (G-CSF), interferons, imiquimod and cellular membrane fractions from bacteria. Others include IL-2, IL-7, IL-12, various chemokines, synthetic cytosine phosphate-guanosine (CpG) oligodeoxynucleotides and glucans.

Immunotherapies designed to elicit or amplify an immune response are classified as activation immunotherapies, while immunotherapies that reduce or suppress are classified as suppression immunotherapies. The term "immunotherapy" herein refers in particular to activation immunotherapy.

The immunotherapy may be for example a cell-based immunotherapy in particular a dendritic cell-based pump-priming or vaccination, a T-cell adoptive transfer, an autologous immune enhancement therapy, or may involve the use of immune checkpoint inhibitor(s) (ICI(s)), of a toxoid, or of an oncolytic virus-based immunotherapeutic agent.

Cell-based immunotherapies may be used for treating cancer. Immune effector cells such as lymphocytes, macrophages, dendritic cells, natural killer cells, and cytotoxic T lymphocytes work together to defend the body against cancer by targeting abnormal antigens expressed on the surface of tumor cells. Vaccine-induced immunity to COVID-19 relies mostly on an immunomodulatory T-cell response.

Dendritic cells (DC) may be stimulated to activate a cytotoxic response towards an antigen. This is the mechanism involved in the context of dendritic cell-based pump-priming or vaccination. Dendritic cells, a type of antigen-presenting cell, are harvested from the person needing the immunotherapy. These cells are then either pulsed with an antigen or tumor lysate or transfected with a viral vector, causing them to display the antigen. Upon transfusion into the person, these activated cells present the antigen to the effector lymphocytes (CD4+ helper T cells, cytotoxic CD8+ T cells and B cells). This initiates a cytotoxic response against tumour cells expressing the antigen (against which the adaptive response has now been primed).

T-cell adoptive transfer *in vitro* cultivates autologous, extracted T cells for later transfusion. Alternatively, genetically engineered T cells are created by harvesting T cells and then infecting the T cells with a retrovirus that contains a copy of a T cell receptor (TCR) gene that is specialised to recognise tumour antigens. The virus integrates the

receptor into the T cells's genome. The cells are expanded non-specifically and/or stimulated. The cells are then reinfused and produce an immune response against the tumour cells, for example refractory stage IV metastatic melanomas, advanced skin cancer and lung cancer. Whether T cells are genetically engineered or not, before re-infusion, lymphodepletion of the recipient is required to eliminate regulatory T cells as well as unmodified, endogenous lymphocytes that compete with the transferred cells for homeostatic cytokines. Lymphodepletion may be achieved by myeloablative chemotherapy, to which total body irradiation may be added for greater effect. Clinical responses to adoptive transfer of T cells were observed in patients with metastatic melanoma resistant to multiple immunotherapies.

The immunotherapeutic agent may also be an immune checkpoint inhibitor (also herein identified as a "checkpoint inhibitor" or "ICI"). Anti-PD-1/PD-L1 and anti-CTLA-4 antibodies are the two types of checkpoint inhibitors currently available to patients. The approval of anti-cytotoxic T-lymphocyteassociated protein 4 (CTLA-4) and anti-programmed cell death protein 1 (PD-1) antibodies for human use has already resulted in significant improvements in disease outcomes for various cancers. Although these molecules were originally discovered as molecules playing a role in T cell activation or apoptosis, subsequent preclinical research showed their important role in the maintenance of peripheral immune tolerance. Immune checkpoint inhibitors are approved to treat some patients with a variety of cancer types, comprising skin cancer including melanoma, breast cancer, bladder cancer, cervical cancer, colon cancer, head and neck cancer, liver cancer, lung cancer, renal cell cancer, stomach cancer, rectal cancer, Hodgkin lymphoma and any solid tumor that is not able to repair errors in its DNA that occur when the DNA is copied.

The immunotherapeutic agent may also be an oncolytic virus-based immunotherapeutic agent or a toxoid.

Autologous immune enhancement therapy may also be used in combination with "compound (a)". This method uses a person's own peripheral blood-derived natural killer cells, cytotoxic T lymphocytes, epithelial cells and other relevant immune cells which are expanded in vitro and then re-infused.

### *Anti-cancer agents*

In the context of the present invention, the additional therapeutic agent ("compound (b)") may be an anticancer agent.

In the context of cancer, a conventional treatment of cancer is chemotherapy. The chemotherapeutic agent may be selected for example from an alkylating agent, a platinum coordination complex, a cytotoxic antibiotic, an antimetabolite, a taxane, a topoisomerase inhibitor, a vinca alkaloid and an intercalating agent.

The anti-cancer agent usable in the context of the present invention may be simultaneously an immunotherapeutic agent such as an ICI, in particular an anti-PD-1, anti-PD-L1 or anti-CTLA-4 antibody; a CAR-T cell; a toxoid; or an oncolytic virus-based immunotherapeutic agent.

In a particular aspect, the anticancer agent is selected for example from an antimetabolic agent, an alkylating agent, a vinca-alkaloid, an intercalating agent, a tyrosine kinase inhibitor (TKI), an antibody or a fragment thereof, an antibody-drug conjugate ("ADC"), an anti-angiogenic agent, an immune check point inhibitor, a CAR-T cell, a toxoid, and an oncolytic virus, in particular an oncolytic virus-based immunotherapeutic agent.

Numerous tyrosine kinase inhibitors (TKIs) have been proven to be effective anti-tumor agents and antileukemic agents. Imatinib was developed against chronic myelogenous leukemia (CML) and later gefitinig and erlotinib aiming at the EGF receptor. Dasatinib is a Src tyrosine kinase inhibitor that is effective both as a senolytic and as therapy for CML. Sunitinib, an inhibitor of the receptors for FGF, PDGF and VEGF is also based on early studies on TKIs aiming at VEGF receptors. Signal transduction therapy can also be used for inflammatory conditions.

The term "anti-angiogenic agent" designates in particular a VEGF inhibitor, VEGFR inhibitor, TIE-2 inhibitor, PDGFR inhibitor, angiopoietin inhibitor, PKCf3 inhibitor, cyclooxygenase II (COX-2) inhibitor, integrin (alpha-v/beta-3), matrix-metalloproteinase 2 (MMP-2) inhibitor or matrix-metalloproteinase 9 (MMP-9) inhibitor.

In a particular aspect, the anti-angiogenic agent is a compound having the following formula :

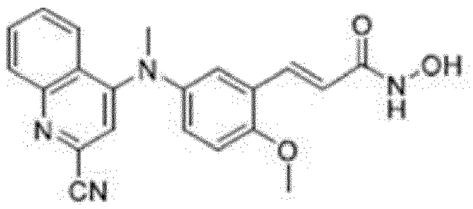

,

also herein identified as "NI-313".

In the context of the present invention, the term "antibody" (or "immunoglobulin") designates any kind of antibody such as a monoclonal antibody, a multispecific antibody (i.e. an antibody comprising a first antigen binding site and at least one second different antigen binding site; e.g. a bispecific antibody) or a single chain antibody. This term also covers as any

(functional) fragment thereof.

An typical antibody consists of a glycoprotein comprising at least two heavy (H) chains and two light (L) chains inter-connected by disulfide bonds. Each heavy chain comprises a heavy chain variable region (or domain) (abbreviated herein as VH) and a heavy chain constant region (hereafter CH). Heavy chains are classified as gamma, mu, alpha, delta, or epsilon, and define the antibody's isotype as IgG, IgM, IgA, IgD, and IgE, respectively. The heavy chain constant region of the immunoglobulin IgG, IgD, and IgA ($\gamma$, $\delta$ and $\alpha$ chains respectively) comprises three domains (CH1, CH2, and CH3) and a hinge region for added flexibility, and the heavy chain constant region of the immunoglobulin IgM and IgE contains 4 domains (CH1, CH2, CH3, and CH4). The antibody of the invention can be of the IgG, IgM, IgA, IgD, and IgE isotype, depending on the structure of its heavy chain. However, in a preferred embodiment, the antibody of the invention is of the IgG isotype, i.e., its heavy chain is of the gamma ($\gamma$) type.

IgG antibodies are classified in four distinct subtypes, namely IgG1 IgG2, IgG3 and IgG4 in the order of their abundance in serum (IgG1 being the most abundant). The structure of the hinge regions in the $\gamma$ chain gives each of these subtypes its unique biological profile (even though there is about 95% similarity between their Fc regions, the structure of the hinge regions is relatively different).

The antibody of the invention can be of the IgG1, IgG2, IgG3 or IgG4 subtype. However, in a preferred embodiment, the antibody of the invention is of the IgG1 subtype.

Each light chain comprises a light chain variable region (abbreviated herein as VL) and a light chain constant region comprising only one domain, CL. There are two types of light chain in mammals: the kappa ($\kappa$) chain, encoded by the immunoglobulin kappa locus on chromosome 2, and the lambda ($\lambda$) chain, encoded by the immunoglobulin lambda locus on chromosome 22. In a preferred embodiment, the antibody of the invention has a Kappa light chain. The VH and VL regions can be further subdivided into regions of hypervariability, termed "Complementarity Determining Regions" (CDR), which are primarily responsible for binding an epitope of an antigen, and which are interspersed with regions that are more conserved, termed "Framework Regions (FR). Each VH and VL is composed of three CDRs and four FRs, arranged from amino -terminus to carboxy-terminus in the following order: FR1, CDR1, FR2, CDR2, FR3, CDR3, FR4. The functional ability of the antibody to bind a particular antigen depends on the variable regions of each light/heavy chain pair, and is largely determined by the CDRs. The variable region of the heavy chain differs in antibodies produced by different B cells, but is the same for all antibodies produced by a single B cell or B cell clone (or hybridome).

By contrast, the constant regions of the antibodies mediate the binding of the immunoglobulin to host tissues or factors, including various cells of the immune system (e.g. effector cells) and the first component (Clq) of the classical complement system.

As used herein, the term "antibody fragments" intends to designate Fab, Fab', F(ab')2, scFv, dsFv, ds-scFv, single chain antibody, dimers, minibodies, nanobodies, diabodies, and multimers thereof and bispecific antibody fragments. Antibodies can be fragmented using conventional techniques. Various techniques have been developed for the production of antibody fragments. Traditionally, these fragments were derived via proteolytic digestion of intact antibodies. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, nanobodies, diabodies, bispecific antibody fragments and other fragments can also be synthesized by recombinant techniques. Typically, the antibody fragment of the invention is a functional fragment, i.e. an antibody fragment capable of binding and preferably inhibiting or neutralizing the activity of a molecule of interest as does the antibody it is deriving from.

In another particular embodiment, the antibody of the invention is a monoclonal antibody.

A "monoclonal antibody", as used herein, designates an antibody arising from a nearly homogeneous population of antibodies. More particularly, the antibodies of a given subject are identical except for a few possible naturally-occurring mutations which can be found in minimal proportions. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is generally characterized by heavy chains of one and only one isotype and subtype, and light chains of only one type. In addition, in contrast with preparations of polyclonal antibodies, each monoclonal antibody is directed to a single epitope of an antigen.

To produce monoclonal antibodies, antibody producing cells (lymphocytes) can be harvested from an immunized animal as described above and fused with myeloma cells by standard somatic cell fusion procedures thereby immortalizing these cells and yielding hybridoma cells. Such techniques are well known in the art (e.g. the hybridoma technique originally developed by Kohler and Milstein (1975)) as well as other techniques such as the human B-cell hybridoma technique, the EBV-hybridoma technique to produce human monoclonal antibodies, and screening of combinatorial antibody libraries. Hybridoma cells can be screened immunochemically for production of antibodies specifically reactive with the target polypeptide(s) so that only monoclonal antibodies binding to said polypeptide(s) are isolated. The antibody or a fragment thereof of the invention may be a human, chimeric, humanized, murine, CDR-grafted, phage-displayed, bacteria-displayed, yeast-displayed, transgenic-mouse produced, mutagenized, or randomized antibody or fragment.

A chimeric antibody is a molecule in which different portions are derived from different animal species, such as those having a variable region derived from a murine monoclonal antibody (mAb) and a human immunoglobulin constant region.

Humanized forms of antibodies of the invention are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, framework region (FR) residues of the human immunoglobulin (recipient antibody) are replaced by corresponding non-human residues of the donor antibody. Furthermore, humanized antibodies may comprise residues that are not found in the recipient antibody or in the donor antibody. In general, the humanized antibody may comprise substantially all of at least one, typically two, variable domains, in which all or substantially all of the hypervariable loops correspond to those of a non-human immunoglobulin (donor antibody having the desired specificity, affinity, and capacity) and all or substantially all of the FRs are those of a human immunoglobulin sequence. Methods for humanizing non-human antibodies have been described in the art. Preferably, a humanized antibody has one or more amino acid residues introduced into it from a source, which is non-human. These non-human amino acid residues are often referred to as "import" residues, which are typically taken from an "import" variable domain. Humanization may be essentially performed by substituting hypervariable region sequences for the corresponding sequences of a human antibody. Accordingly, such humanized antibodies are chimeric wherein substantially less than an intact human variable domain has been substituted by the corresponding sequence from a non-human species. In practice, humanized antibodies are typically human antibodies in which some hypervariable region residues and possibly some FR residues are substituted by residues from analogous sites in rodent antibodies. Other methods generally involve conferring donor CDR binding affinity onto an antibody acceptor variable region framework. One method involves simultaneously grafting and optimizing the binding affinity of a variable region binding fragment. Another method relates to optimizing the binding affinity of an antibody variable region. The antibody or fragment thereof of the invention may be administered in their "naked" or unconjugated form, or may have other agents conjugated to them, such as drug, toxin or radioactive atom.

The "antibody" is for example an anti-PD-1 such as for example only pembrolizumab, nivolumab, cemiplimab or dostarlimab; an anti-PD-L1 such as for example only atezolizumab, avelumab or durvalumab; or an anti-CTLA-4 antibody such as ipilimumab for example.

The term "antibody-drug conjugate" ("ADC") designates in particular an ADC compound such as for example trastuzumab emtansine, fam-trastuzumab deruxtecan-nxki (Enhertu®), trastuzumab duocarmazine, disitamab vedotin, ladiratumab vedotin (also known as SGN-LIV1A), depatuxizumab mafodotin, SN-38, anti-nectin-4, TROP-2, or a pharmaceutically acceptable polymorph, enantiomer, stereoisomer, salt, solvate or tautomer of any of the foregoing.

Cancer immunotherapy attempts to stimulate the immune system to destroy tumours. Its efficacy is enhanced by 20-30% when cell-based immunotherapy is combined with conventional treatment methods such as chemotherapy or radiotherapy.

***Medications used to treat infections***

Specific medications used to treat infections include antibiotics, antivirals, antifungals and antiparasitic (including antiprotozoals and anthelminthics). Depending on the infection's type, the skilled person will know the one to select and used as "compound (b)".

***Medications used to treat inflammation or inflammatory diseases***

Specific medications used to treat inflammation include non-steroidal anti-inflammatory drugs (NSAIDs), glucocorticoids, and a signal transduction therapy as described herein above.

Depending on the inflammation's symptoms or inflammatory diseases' type, the skilled person will know the one to select and used as "compound (b)".

***Medications used in the context of a neurodegenerative disease***

In a particular aspect of the present invention, the additional therapeutic agent ("compound (b)") may be a therapeutic agent such as a microtubule stabilizing drug, used to prevent the appearance or development of a neurodegenerative disease as identified in the "definition" section (Amyotrophic lateral sclerosis ("ALS"), multiple sclerosis, Parkinson's disease, Alzheimer's disease, Huntington's disease, Batten disease, multiple system atrophy, and prion diseases such as Creutzfeld-Jakob's disease), in particular Parkinson's disease or Alzheimer's disease.

The current treatments for neurodegenerative diseases are mostly symptomatic without affecting the underlying

cause of disease.

### *PTPs*

The additional therapeutic agent may be a PTP or a nucleic acid sequence (DNA or mRNA) or genetic construct or vector encoding a PTP. Methods of preparing PTPs are described in WO2017/149118.

In a particular aspect, the PTP is derived or purified from the cancer tumor of the subject ("tumor-associated PTP" or "TA-PTP"). Such a TA-PTP has been identified by inventors as a PTP activating CD8+ T cells.

Genetic vaccination can be performed using a variety of viral vectors, such as vaccinia, pox virus, adenovirus, adeno associated virus, etc., non-viral vectors, such as nucleic acid sequence associated with various lipidic or peptidic compositions, or using pure (e.g., naked or in other words free of any transfection facilitating agent) nucleic acid.

In another preferred aspect, this PTP is used as a cancer vaccine in combination with a corresponding full-length protein or polypeptide, i.e. with a protein or polypeptide canonically translated by the same mRNA, or with an antigen thereof, and/or with exosome(s).

### *Exosomes*

Exosomes are vesicles having a diameter of 30 to 100 nm.

Tumor-derived exosomes produced by tumor cells may be collected and/or purified according to techniques known in the art, such as by centrifugation, chromatography, etc. Preferred techniques have been described in WO00/44389 and in US09/780,748, incorporated herein by reference.

Methods of preparing functionalized exosomes are described in WO2017/149118.

PTPs can be used in combination with exosomes to create a potent immune cancer vaccine [Duvallet E. et al., 2016], in particular with exosomes comprising PTPs. The antigenic epitope derived from PTP may be exposed outside of the exosome, and the PTP is typically contained within the exosome (i.e., attached to the inner side of the membrane or in suspension inside the microvesicle). Typically, the exosome allows efficient transport of the PTP(s) to the dendritic cells and allows efficient cross-presentation of the PTP(s) and antigenic epitope(s) derived therefrom at the dendritic cell surface.

In the (vaccine) composition of the invention, PTPs are present in an amount sufficient to elicit a therapeutic response of the immune system of a given subject against a desired target (pathogen, target cell), for example a CD8+ T cells response, typically a CD4+ T cells response, preferably CD4+ and CD8+ T cells therapeutic responses, and prevent or treat, typically control, a disease, for example a cancer.

When the subject is a mammal, preferably a human being, the vaccine composition may typically comprises from 0.1 to 10 mg per kg of body weight of PTPs, optionally together with 0.1 to 5 mg per kg of body weight of microvesicles.

The subject may also be immunized using cell lines transfected *in vitro* with vectors coding PTPs, the cell lines being for selected for the ability to secrete exosomes.

Each of the herein described therapeutic agents may be used as a "compound (b)" in combination with "compound (a)". In a preferred aspect of the invention, the compound (b) is an immune checkpoint inhibitor (ICI), in particular an anti-PD-1, anti-PD-L1 or anti-CTLA-4 antibody. Compound (b) may be any therapeutic compound or therapy herein identified, such as for example the NI-313 compound or a T-cell transfer adoptive therapy.

## THERAPEUTIC USES

Inventors discovered and herein reveal for the first time that a compound of formula (I) ("compound (a)"), in particular the compound identified as "compound 58", can advantageously be used as an immunomodulating, preferably immunostimulating, agent alone or in combination with a distinct therapeutic agent ("compound (b)") as identified in the preceding section.

Thus, inventors herein describe for the first time this compound (a) for use for preventing or treating immunodeficiency or for enhancing a subject's immune response, alone or in combination with a compound (b). In other words, they describe this compound (a) alone or in combination with a compound (b) for use for treating an immunodeficient subject.

In a particular aspect, inventors describe this compound (a) alone or in combination with a compound (b) for use in the context of a targeted therapy aimed at overcoming REGγ-mediated immunosuppression.

The present description also relates to the use of a product of the invention as herein described [i.e., compound (a), compositions and combinations comprising such a compound (a)] for preparing a composition, typically a pharmaceutical composition, in particular a medicament, for example a vaccine composition, for preventing or treating a disease in a subject. A typical composition or combination is for use in a mammal, in particular a human being.

Thus, inventors also herein describe combinations of compounds and compositions, typically pharmaceutical

compositions, for use as a medicament.

The combinations are combinations of (a) a compound of formula (I) ("compound (a)"), in particular the compound identified as "compound 58", and (b) a distinct therapeutic agent ("compound (b)"), preferably an ICI.

The compositions comprise a compound of formula (I) ("compound (a)"), in particular the compound identified as "compound 58", and a pharmaceutically acceptable carrier. In a preferred aspect, the compositions in addition comprise a distinct therapeutic agent ("compound (b)"), preferably an ICI.

Inventors in addition advantageously herein describe a compound of formula (I) ("compound (a)"), in particular the compound identified as "compound 58", combinations of compounds comprising such a compound (a) and preferably an additional therapeutic agent ("compound (b)"), as well as pharmaceutical compositions comprising such a compound (a) and a pharmaceutically acceptable carrier, preferably together with a distinct additional therapeutic agent ("compound (b)"), and optionally a pharmaceutically acceptable carrier, for use for preventing or treating a disease or symptom(s) of a disease.

As explained herein above, the disease (or symptom) may be selected from for example a cancer, an inflammatory disease or inflammation which may be induced by a cancer or an infection or may be involved in neurodegeneration; and an infectious disease which may be a primary or secondary infection and which is preferably a viral or bacterial infection, preferably an infectious disease which is not induced by M. tuberculosis.

The cancer be any kind of cancer described in the "definition" section such as for example a head and neck cancer, in particular head and neck squamous cell carcinoma, a thyroid carcinoma, a esophageal carcinoma, a breast cancer in particular a breast invasive carcinoma, a lung cancer in particular a non-small-cell lung cancer, a lung adenocarcinoma or a lung squamous cell carcinoma, a liver hepatocellular carcinoma, a cholangiocarcinoma, a renal cancer in particular a kidney renal clear cell carcinoma, a kidney chromophobe or kidney renal papillary cell carcinoma, a bladder urothelial carcinoma, a prostate cancer in particular a prostate adenocarcinoma, a skin cancer in particular a skin cutaneous melanoma, a rectum adenocarcinoma, a uterine corpus endometrial carcinoma, a colon or colorectal cancer in particular a colon adenocarcinoma, a gastric cancer in particular a stomach adenocarcinoma, an oral squamous cell carcinoma, a multiple myeloma, and a pancreatic cancer. It is preferably a cancer wherein cancerous tumor or tumor cells overexpress "REGγ".

Herein described in particular is a compound, composition or combination according to the invention for use for treating a cancer in a subject, in particular a cancer wherein, compared to the healthy cells of the subject, the tumor cells of the subject overexpress REGg. The cancer is preferably a metastatic cancer or a cancer resistant and/or refractory to standard-of-care treatment, in particular to immunotherapy. The disease may also be a neurodegenerative disease such as Amyotrophic lateral sclerosis ("ALS"), multiple sclerosis, Parkinson’s disease, Alzheimer’s disease, Huntington’s disease, Batten disease, multiple system atrophy, and prion diseases such as Creutzfeld-Jakob’s disease. In a particular aspect, the disease is not a neurodegenerative disease.

Typical symptoms which may be prevented or treated thanks to the present invention are for example inflammation’s or immune disease’s symptoms as herein above described in the "definition" section.

## TREATMENT METHODS

An object of the invention also relates to a method of producing an immune response in a subject, typically a method of vaccinating a subject, against a specific target, typically an antigen, for example a tumor antigen or cancer cell, the method comprising administering to said subject a product [i.e., compound (a), composition or combination comprising such a compound (a)] according to the invention.

The detection of a therapeutic immune response can be easily determined by the skilled person thanks to technologies such as ELISA, ELISPOT, delayed type hypersensitivity response, intracellular cytokine staining, and/or extracellular cytokine staining.

Another object of the invention is a method of preventing or treating a disease or symptom as herein described, preferably a cancer, in a subject, the method comprising administering to said subject a product [i.e., compound (a), composition or combination comprising such a compound (a)] according to the invention.

As used herein, the terms "combination" or "combination therapy" refer to the administration of each therapeutic agent of the combination therapy of the invention, either alone or in the form of a pharmaceutical composition or medicament, either sequentially, concurrently, or simultaneously.

As used herein, the term "sequential" or "sequentially" refers to the administration of each therapeutic agent of the combination therapy of the invention, either alone or in a medicament, one after the other, wherein each therapeutic agent can be administered in any order. Sequential administration may be particularly useful when the therapeutic agents in the combination therapy are in different dosage forms, for example, one agent is a tablet and another agent is a sterile liquid, and/or the agents are administered according to different dosing schedules, for example, one agent is administered daily, and the second agent is administered less frequently such as weekly.

As used herein, the term "concurrently" refers to the administration of each therapeutic agent in the combination

therapy of the invention, either alone or in separate medicaments, the second therapeutic agent being administered immediately after the first therapeutic agent, and the therapeutic agents being administered in any order. In a preferred embodiment the therapeutic agents are administered concurrently.

As used herein, the term "simultaneous" refers to the administration of each therapeutic agent of the combination therapy of the invention in the same medicament. As will be understood by those skilled in the art, the combination therapy may be usefully administered to a subject during different stages of their treatment.

Thus, inventors in particular herein describe a combination or composition for use of the invention, wherein (a) and (b) are formulated for simultaneous, concurrent or sequential administration by the same administration route or by different administration routes.

***Routes of administration***

The herein described products capable of inducing a therapeutic immune response may be administered *in vivo* to any subject in need thereof, typically mammalian subjects, in particular human subjects.

Administration or vaccination can be performed through various routes, such as by systemic injection, e.g., intravenous, intra-muscular, intra-peritoneal, intra-tumoral, sub-cutaneous, intradermal etc. Various vector delivery devices or techniques well known by the skilled person in the art may be used for genetic vaccination, including gene gun or electroporation.

***Posology***

The compound of formula (I) of the invention ("compound (a)") is typically used, alone or in combination with a distinct therapeutic agent, at an "effective dosage" "therapeutically effective amount" which will be determined by the skilled person depending on the disease or symptom to be prevented or treated.

As used herein, an "effective dosage" or "effective amount" of a compound, combination or pharmaceutical composition is an amount sufficient to affect any one or more beneficial or desired outcomes, including biochemical, histological and/or behavioral symptoms, of the disease, its complications and intermediate pathological phenotypes presenting during development of the disease. For therapeutic use, a "therapeutically effective amount" refers to that amount of a compound or combination being administered which will relieve to some extent one or more of the symptoms of the disorder being treated.

In reference to the treatment of cancer, a therapeutically effective amount refers to that amount which has the effect of reducing the size of the tumor, inhibiting (that is, slowing to some extent, preferably stopping) tumor metastasis, inhibiting to some extent (that is, slowing to some extent, preferably stopping) tumor growth or tumor invasiveness, relieving to some extent (or, preferably, eliminating) one or more signs or symptoms associated with the cancer, decreasing the dose of other medications required to treat the disease, enhancing the effect of another medication, and/or delaying the progression of the disease in a patient.

An effective dosage can be administered in one or more administrations. For the purposes of this invention, an effective dosage of drug, compound, combination or pharmaceutical composition is an amount sufficient to accomplish prophylactic or therapeutic treatment either directly or indirectly. As is understood in the clinical context, an effective dosage of a drug, compound or pharmaceutical composition may or may not be achieved in conjunction with another drug, compound, combination or pharmaceutical composition.

The "compound (a)" of the invention is typically present in the combination or composition herein described at a dose of about 1 mg to about 200 mg, preferably about 1 mg to about 50 mg, more preferably about 10 mg.

**KITS**

Also herein described is a pharmaceutical kit for use in the prevention or treatment of a disease herein described, or of symptoms thereof. As explained in the "definition" section, the disease is typically selected from a cancer, an inflammation, a neurodegenerative disease and an infectious disease (preferably an infectious disease which is not induced by *M. tuberculosis*). This kit typically comprises i) a "compound (a)", in particular "compound 58", and ii) an additional distinct therapeutic agent also herein identified as "compound (b)" such as for example an immunotherapeutic agent, an anti-cancer agent, a therapeutic agent used to treat an infection, an inflammation or inflammatory disease, a medication used to prevent or treat a neurodegenerative disease and any combination thereof, in particular immunotherapeutic agent and/or an anti-cancer agent, preferably an immune checkpoint inhibitor (ICI) such as an anti-PD-1, anti-PD-L1 or anti-CTLA-4 antibody, in distinct containers.

A particular kit comprises for example i) a "compound (a)", in particular "compound 58", and ii) the NI-313 compound, in distinct containers.

A particular kit comprises for example i) a "compound (a)", in particular "compound 58", and ii) one or more PTPs

that have been induced by the compound 58, in distinct containers.

A particular kit comprises for example i) a "compound (a)", in particular "compound 58", and ii) exosomes, in distinct containers.

The kits described herein may be particularly suitable for administering different dosage forms, for example, oral and parenteral, for administering the separate active (in particular therapeutic) agents of the combination or compositions at different dosage intervals, or for titrating the active (in particular therapeutic) agents of the combination or compositions against one another. To assist compliance, the kit typically includes directions for administration and may be provided with a memory aid. The kit may further comprise other materials that may be useful in administering the medicaments, such as diluents, filters, IV bags and lines, needles and syringes, and the like.

Throughout this application, various references describe the state of the art to which this invention pertains. The disclosures of these references are hereby incorporated by reference into the present disclosure.

Others features and advantages of the invention appear in the following examples and figures which are given for purposes of illustration and should not be interpreted in any way as limiting the scope of the present invention.

## FIGURES

**Figure 1A. Chemical structure of compound 58 (also herein identified as "58" or "NSC 214009")**

**Figure 1B. other possible compounds of interest according to the Invention.**

**Figure 2. Effect of the compound 58 on the three catalytic activities of the 20S and REGy-20S proteasome.**

The effect of the compound 58 on the tryspin-like (A), caspase-like (B) and chemotrypsin like (C) activities of the proteasome was studied with specific peptidic substrates. Results are shown as percentage of activity with the DMSO conditions set as 100% + SEM in at least three independent experiments. Two-way ANOVA with multiple comparison. **p<0.05, **p<0.01, ***p<0.001, ****p<0.0001.*

**Figure 3. Thermal shift assay of compound 58 with REGγ and 20S.**

(A) Thermal shift assay of compound 58 with REGγ, raw data (top) and 1st derivative of the data (bottom) are represented. (**B**) Thermal shift assay of the compound 58 with 20S, raw data (top) and 1st derivative (bottom) are represented.

**Figure 4. Effect of compound 58 on the degradation of KH-52 peptide.**

(A) Degradation rate of KH-52 by either 20S (dashed lines) or REGy-20S proteasome (plain lines). Line represent the calculation of a linear-regression model from four independent experiments. (B) Slope values of the conditions with DMSO and 50μM of compound 58 with the REGγ-proteasome are represented as mean ± SEM of four independent experiments. Comparison of the slope was performed using graphpad PRISM software with ANCOVA test, *ns, not significant.*

**Figure 5. MTT Assays with compound 58 on MCA-205 and A375 cells**

(A) MTT assay with wild type (MCA) and REGγ-KO MCA-205 cells (C13). (B) MTT assay with wild type (A375) and REGγ-KO A375 cells (1375 CXI). Data are shown as the mean of three independent experiments + SEM. Nonlinear regression for each dataset was calculated with Graphpad PRISM software.

**Figure 6. Effect of the compound 58 on antigen presentation and PD-L1 surface expression on MCA205-Globi.**

Expression of H2Kb molecules bound to SL8 (SIINFEKL) peptide (A), H2Kb molecules (B), H2Db molecules (**C**) and PD-L1 (**D**) at the cell surface. Left panel, cytometry plots representative of three independent experiments. Right panel, MFI of at least three independent experiments are represented as mean ± SEM of ratio over DMSO. Statistics test used is One-way ANOVA with multiple comparisons. **p<0.05, **p<0.01, ***p<0.001, ****p<0.0001.*

**Figure 7. Effect of the compound 58 on the activation of OT-1 T cells by MCA205-Globi cells.**

(A) Expression of activation markers CD25 and CD69 at the surface of OT-1 T cells (bottom graph) and frequence of $CD25^+CD69^+$cells following treatment with compound 58. (**B**) Micro-Flow imaging (MFI) of CD25 (right) and CD69 (left)

markers as fold over DMSO. (**C**) Frequencies of effector T cells (CD44+CD62L-) and Central memory T cells (CD44+CD62L+) after treatment with compound 58. (**D**) Perforin α expression of OT-1 T cells after treatment with compound 58. Median of five biologicallyindependent samples, representative of three independent experiments. One-way ANOVA with multiple comparison. *$p<0.05$, $**p<0.01$, $***p<0.001$, $****p<0.0001$.*

## EXAMPLES

The following non-limiting examples are provided to further illustrate certain teachings provided by the present disclosure. Those of skill in the art, in light of the present application, will appreciate that various changes can be made in the specific embodiments that are illustrated in the present Examples without departing from the spirit and scope of the present teachings.

## EXAMPLE 1

## MATERIALS AND METHODS

### Screening of Compounds

The 3 chemical libraries tested were obtained from the NIH : The *Natural Products* set (NP.V) consisting of 390 compounds, the *Oncology Approved Drugs* set (AOD.IX) consisting of 147 FDA-approved compounds and the *Diversity* set (DIV.VI) consisting of 1584 compounds. Libraries were prepared and screened with a Biomek FXP workstation in 384 well plates. Compounds activity was assessed using a fluorescence activity test of the proteasome using the Bz-VGR-AMC fluorogenic substrate (#BML-BW9375-0005, Enzo Life Sciences). 50μM of the fluorogenic substrate was used with 20μM of the tested compound, and 2nM of 20S human proteasome (#E-360-050, Biotechne) in presence or absence of 50nM of human REGγ (#E-384-MTO, Biotechne). After preparation of the mix, plates were incubated for 2h at 37°C before being read on a Spectramax i3 microplate reader from Molecular devices (Excitation wavelength : 345nm ; Emission wavelength : 445nm). For each plate, inventors had 3 controls, one with only the Substrate + DMSO (*VGR*), one with Substrate + DMSO + 20S (20S) and a control with Substrate + DMSO + 20S + REGγ (REG). Each compound was tested in quadruplicate either with or without REGγ.

### Hits selection

After QC verification, each plate was normalized to the mean of the *VGR* control. For every quadruplicate the most different value from the mean was eliminated and the fluorescence mean for each compound was calculated on the 3 remaining values. The percentage of inhibition of a compound was calculated according to, as follows :

$$\%Inhibition\ (compound) = \frac{Mean_{REG,plate} - Mean_{compound}}{Mean_{REG,plate} - Mean_{20S,plate}} x100,$$

for the condition with REG□.

Using the condition with REGγ, inventors selected compounds with a mean fluorescence value inferior to $Mean_{REG,plate}$ - *3xMAD*($Mean_{REG,plate}$), where *MAD* is the Mean Adjusted Difference. Using the control condition without REGγ, they then eliminated compounds that were inhibiting the 20S proteasome, using the 95% interval of confidence of the 20S control for each plate. Finally, they selected compounds from this list that had at least a 25% inhibition of the 20S-REGD complex. They identified 81 hits that were then tested on 2 confirmatory screens. The first one aimed to eliminate false-positive inhibitors. They tested the compounds on the 20S-REGγ complex at 10, 20 and 40μM for 90min with a kinetic assay. Fluorescence was measured every minute with a FLUOstar plate reader (BMG Labtech). The second confirmatory screen was performed accordingly, but compounds were tested only on the 20S proteasome.

**Proteasome Activity Assay** - The activity of the 20S and 20S-REGγ proteasomes was followed by fluorescence using the Bz-VGR-AMC (#BML-BW9375-0005, Enzo Life Sciences) substrate at 50μM for tryspin-like activity, the Suc-LLVY-AMC (#BML-P802-0005, Enzo Life Sciences) substrate at 100μM for the chemotrypsin-like activity and the Z-LLE-AMC (#BML-ZW9345-0005, Enzo Life Sciences) substrate at 100μM for the caspase-like activity. Substrates were incubated with 0,5nM of human 20S ((#E-360-050, Biotechne) and 12,5nM of human REGγ or 2nM of human REGαβ with or without compounds at different concentrations. Purified human REGγ and REGGαβ were generously provided by Julien Marcoux and Marie-Pierre Bousquet-Dubouch. Human REGγ and REGαβ were purified according to *Le Feuvre et al., 2009* from plasmids kindly provided by Olivier Coux. The activity assay was performed in 96 well plates in a final volume of 80μL. Plates were read every minute for 90 minutes with a FLUOstar plate reader (BMG Labtech). To detect fluorescence,

the excitation wavelength was set to 355nm and emission wavelength at 460nm.

**Cell lines and culture**

The MCA205-Glob-SL8-intron cell line was obtained from MCA205 fibrosarcoma cell line and was modified to constitutively express the SIINFEKL epitope contained within an intron of the human β-globin gene, as described in (Duvallet *et al., 2016*). Human melanoma A375 cell line was obtained from ATCC (n°CRL-1619). The MCA205 c13 and A375 c.XI cell lines, both KO for PSME3 gene were obtained using the CRISPR/Cas9 system as described in Boulpicante *et al.* All cells were cultured at 37°C in a 5% $CO_2$ atmosphere. MCA205, MCA205-Glob-SL8-intron and MCA205 c13 cell lines were cultured in RPMi-1640 (#21870084, Gibco) supplemented with 10% (v/v) decomplemented fetal bovine serum (FBS), 2mM L-glutamine (#A2916801, Gibco), 1mM sodium-pyruvate (#11360070, Gibco) and 1% (v/v) of non-essential amino-acids (#11140050, Gibco). A375 and A375 cXI cell lines were cultured in DMEM (#11960044, Gibco) supplemented with 10% (v/v) of decomplemented FBS and 2mM L-glutamine (#A2916801, Gibco).

**Proteasome Activity Assay**

The activity of the 20S and 20S-REGγ proteasomes was followed by fluorescence using the Bz-VGR-AMC (#BML-BW9375-0005, Enzo Life Sciences) substrate at 50μM for trypsin-like activity, the Suc-LLVY-AMC (#BML-P802-0005, Enzo Life Sciences) substrate at 100μM for the chemotrypsin-like activity and the Z-LLE-AMC (#BML-ZW9345-0005, Enzo Life Sciences) substrate at 100μM for the caspase-like activity. Substrates were incubated with 0,5nM of human 20S ((#E-360-050, Bio-techne) and 12,5nM of human REGγ or 2nM of human REGαβ with or without compounds at different concentrations. Purified human REGγ and REGαβ were generously provided by Julien Marcoux and Marie-Pierre Bousquet-Dubouch. Human REGγ and REGαβ were purified according to Le Feuvre et al., 2009 from plasmids kindly provided by Olivier Coux. The activity assay was performed in 96 well plates in a final volume of 80μL. Plates were read every minute for 90 minutes with a FLUOstar plate reader (BMG Labtech). To detect fluorescence, the excitation wavelength was set to 355nm and emission wavelength at 460nm.

**Thermal Shift Assay**

Thermal shift assay was carried out with nano differential scanning fluorimetry (nanoDSF) using the Tycho NT.6 from Nanotemper and was performed at the I2BC platform (Gif-sur-Yvette). Samples were prepared in a final volume of 25μL in a 96-well plate prior to be loaded on the Tycho capillaries. Proteins were diluted in activity buffer (20mM Tris-HCl pH 7.4 and 20mM NaCl). After optimisation, REGγ concentration was set to 500nM and 20S concentration to 50nM. Compounds concentrations were determined as ratios to protein concentration and to stay below 0,5% of DMSO; accordingly, compounds with REGγ were tested at 40X (20μM, 0,4% DMSO) and at 100X (5μM, 0,1% DMSO) with 20S. A thermal ramp was applied from 20 to 95°C and fluorescence was followed at 350nm and 330 nm, measuring intrinsic fluorescence of the proteins from tryptophan and tyrosine residues. Binding was detected as a shift in the inflection temperature ($T_i$) compared to the DMSO control.

**Peptide degradation assay with Fluorescamine**

Degradation of the 52 amino acid peptide KH-52: KVNVDEVGGEALGRLVSRLQDRSIINFEKLFKETNRNWA CGDREDSWVSDRH, Boulpicante *et al.*) by the 20S proteasome was followed by amino-acid group generation using Fluorescamine (#F9015, Sigma-Aldrich). The peptide substrate was incubated at 100μM with 25nM 20S, with or without 160nM REGγ and with different concentration of the tested compounds or DMSO. Assays were performed in 100μL in phosphate buffer (pH 6.8) and incubated at 37°C in a water bath for 8 hours. After 4, 6 and 8 hours, 22μL of sample were retrieved and added to 88μL of phosphate buffer before being stored at - 20°C prior to revelation. For the revelation, samples were thawed and filtered in a centrifuge 20min at 4°C 1kDa filters (#OD003C34, Pall Corporation). To avoid any amino group contamination, filtered were washed in phosphate buffer before use. 20μL of filtered-sample were plated in triplicates in a black 96-wells plate (#237108, Thermo Scientific) and 10μL of fluorescamine (1mg/mL, in 100% acetone) was added to the samples. The plate was placed on agitation for 1min before the reaction was stopped by adding 200μL of water to each well. The fluorescence intensity was measured using a FluoSTAR plate reader (BMG Labtech) with excitation wavelength at 355nm and emission wavelength at 460nm.

**MTT Assays**

$2,5\times10^4$ of MCA cells or $5\times10^4$ of MCA c13 cells were plated in quadruplicates in clear 96-well plates and incubated overnight. The next day, cells were with various concentration of the tested compounds for 24h. After treatment,

media was carefully removed before adding 50μL of new media and 10μL of tetrazolium salt solution (5mg/mL in PBS) and homogeneizing the well by pipetting up and down. The plate was then incubated for 2 hours at 37°C. After incubation, plate was centrifuged 5min at 1500rpm before adding 150μL of DMSO to each well to solubilize the formazan cristals. The plate was incubated on agitation for 10min and absorbance was read at 544nm using a EL808 (Biotek) plate reader.

**MHC-I expression and antigen presentation in MCA205-Glob-SL8-intron**

$2\times10^5$ of MCA205-Glob-SL8-intron cells were plated in 6-well plates and incubated overnight. The next day cells were treated with different concentrations of drugs for 24h. After treatment, cells were harvested with trypsin before being stained with Zombie Aqua (#423102, Biolegend) and antibodies presented in Table 1 following the protocol provided by the supplier. Samples were acquired on a FACS LSRFortessa X20 (BD Biosciences) and data was analysed with FlowJo (V10) software (TreeStar, Inc.).

Table 1: Panels of antibodies for the study of surface expression of MHC-I molecules and SIINFEKL presentation in MCA205-Glob-SL8-intron.

| Panel | Marker | Fluorochrome | Clone | Dilution | Reference | Furnisher |
|---|---|---|---|---|---|---|
| 1 | $H2-K^b$ | PE | AF6-88.5 | 1:100 | 116508 | Biolegend |
| | $H2-D^b$ | APC | KH95 | 1:100 | 111513 | Biolegend |
| 2 | PD-L1 | PE | MIH7 | 1:80 | 155404 | Biolegend |
| | $H2-K^b$ bound to SIINFEKL | APC | 25-D1.16 | 1:60 | 141606 | Biolegend |

**Co-culture of OT-1 cells with MCA205-Glob-SL8-intron**

MCA205-Glob-SL8-intron were plated at $1\times10^5$ cells in 12-well plates and incubated overnight. The next day, cells were treated with different concentrations of drugs for 24 hours. After treatment, cells were harvested with trypsin, washed twice with OT-1 Media (RPMI-1640, 10% FBS, 2mM L-glutamine, 50μM of 2-mercaptoéthanol, 100 IU/mL of Penicillin G, 100 mg/mL streptomycin), and each condition was counted with a KOVA slide and Trypan-blue. For each tested condition $2\times10^5$ of treatedcells were plated in a 96-wells plate. The same day, lymph nodes from an OT1 mouse (C57B1/6J TCR-transgenic $CD8^+$ T cells specific for Kb -OVA257-264, obtained from Charles River #642OT1) were harvested. Lymph nodes were dissociated and filtered through a 40 μm filter and red blood cells were lysed with ACK buffer. $8\times10^5$ OT-1 cells were added to the MCA205-Glob-SL8-intron cells (E:T ratio of 4:1) and the plate was incubated overnight at 37°C. The next day, translocation to the golgi was blocked with brefeldin A (#420601, Biolegend) for 3h before cells were stained with Zombie Aqua (#423102, Biolegend) and antibodies described in Table 2, to measure T cell activation. Samples were acquired on a Cytek Aurora (Cytek Biosciences) spectral flow cytometer and data was analysed with FlowJo (V10) software (TreeStar, Inc.).

Table 2: Panel of antibodies for the study of OT-1 CD8+ T-cells activation by MCA205-Glob-SL8-intron.

| Marker | Fluorochrome | Clone | Dilution | Reference | Furnisher |
|---|---|---|---|---|---|
| CD8 | BV421 | 53-6.7 | 1:300 | 100753 | Biolegend |
| TCR-β | PE/Cyanine 5 | H57-597 | 1:300 | 109209 | Biolegend |
| CD25 | PE | PC61 | 1:300 | 102008 | Biolegend |
| CD69 | APC | H1.2F3 | 1:300 | 104514 | Biolegend |
| CD62L | BUV395 | MEL-14 | 1:100 | 740218 | BD Biosciences |
| CD44 | BV605 | IM7 | 1:200 | 103047 | Biolegend |
| Perforin | FITC | S 16009A | 1:400 | 154310 | Biolegend |
| IFNγ | BV650 | XMG1.2 | 1:50 | 505832 | Biolegend |

## RESULTS

### Identification of compounds that inhibit the effect of REGγ on proteasomal protein degradation

Initial efforts to discover proteasome inhibitors were often performed using high-throughput biochemical screens. These methods use purified proteasome and monitor proteasome activity through co-incubation with a fluorescent substrate. Since 1983, many fluorogenic peptide substrates have been synthesized to monitor each of the activities of the proteasome. The most commonly used substrate employ a 7-amino-4-methylcoumarin (AMC) group on the C-terminus. This group has a high degree of fluorescence. To identify compounds capable of inhibiting the effect of REGγ on proteasomal protein degradation, with the aim of restoring or enhancing cancer antigen presentation inventors conducted a high-throughput screening of three different libraries from NIH: the Approved Oncology Drugs Set (147 compounds), the Diversity Set (1584 compounds), and the Natural Products Set (390 compounds). Due to the complexity of conducting large-scale antigen presentation tests, they decided to focus on a specific activity of the 20S proteasome using a fluorescence-based assay. For the degradation assay, they used purified 20S proteasome from human erythrocytes, a purified human REGγ and a fluorescent substrate Bz-VGR-AMC (7-amino-4-methylcoumarin, Enzo), all mixed together for the reaction at 37°C. The Bz-VGR-AMC substrate was employed to measure the Trypsin-like activity of the proteasome. As mentioned earlier, the AMC fluorophore is released upon substrate cleavage by the proteasome, and the resulting fluorescence is directly proportional to the proteolytic activity.

For a molecules to be classified as "Hits" it must exhibit the following properties: it had to inhibit the activation of REGγ on the 20S proteasome and not inhibit the activity of the 20S proteasome alone. To achieve this, the identification process was divided into two steps. First, inventors identified all compounds that reduced proteasome degradation in the presence of REGγ. Then, among these identified compounds, they eliminated those that inhibited the 20S proteasome even in the absence of REGγ. This was accomplished using "control" plates containing the 20S proteasome, substrate, and compounds, but without the regulator.

After conducting the screening and validating the quality control using a custom algorithm, they analyzed the data to select their hits. Following this computational analysis, they chose 81 hits, which were then subjected to two consecutive counter-screens. The first counter-screen was conducted to confirm that the selected molecule indeed inhibited the 20S-REGγ complex, and the second aimed to ensure that it did not excessively inhibit the 20S proteasome alone.

As a result of these two counter-screens, inventors identified 5 molecules of the 81 first that inhibit the effect of REGγ on proteasomal protein degradation without inhibiting the 20S core alone. As the 5 compounds did not necessarily possess names, they designated them by the numbers assigned during the counter-screen: 58, 62, 66, 70, and 81.

In order to make the present invention easier to understand, the rest of the results will focus on one of the molecules inventors discovered through high throughput screening, the compound 58 (Figure 1).

### Confirmation of the inhibitory effect of compound 58 *in vitro.*

To validate the outcomes of the initial screening test and confirm the identification of Compound 58 as an inhibitor of REGγ's regulatory influence on the 20S proteasome, inventors conducted *in vitro* protein degradation experiments following the same principles as the initial screening. To assess the impact of compound 58, they performed a dose-dependent analysis of its effect on the distinct proteolytic functions of the 20S proteasome. Their screening primarily focused on how various compounds affected the trypsin-like activity of the 20S core. Additionally, they explored the influence of the compound 58 on the other two proteasomal activities: chymotrypsin-like and caspase-like activities. To measure each of these proteasomal activities within the 20S core, different substrates were incubated with 0.5nM of human 20S in the presence of 12.5nM of human REGγ, both with and without compound 58 at various concentrations. Inventors findings confirmed that the compound 58 exhibited inhibitory effects on the 20S-REGy complex. Specifically, compound 58 displayed a more potent inhibitory effect on the caspase-like and trypsin-like activities compared to the chymotrypsin-like activity. Notably, compound 58 starts inhibiting caspase-like activity and trypsin-like activity at 50 and with a stronger effect at 100 μM (Figures 2A and 2B), whereas it only inhibited chymotrypsin-like activity at 100 μM (Figure 2C). Furthermore, it is important to highlight that compound 58 did not exhibit any inhibitory effect, even at high doses, on the three activities when the 20S proteasome was not in the presence of REGγ.

These findings underscore the value of compound 58 as a tool for modulating proteasomal activities regulated by REGγ.

### Selective binding of molecule 58 to REGγ revealed by Thermal Shift Assay (TSA).

To assess the efficacy of molecule 58 in directly binding to REGγ, inventors conducted a thermal shift assay (TSA). TSA leverages the property of proteins to undergo unfolding at specific temperatures, and the interaction with a ligand can alter the thermal stability, leading to a shift in the unfolding temperature. In their study, inventors examined how

the compound 58 interacted with REGγ and monitored the change in melting temperature (ΔTm). Upon incubation with REGγ, molecule 58 exhibited a substantial increase in ΔTm, indicating a robust interaction (Figure 3A). In contrast, when incubated with the 20S proteasome, there was no significant change in ΔTm (Figure 3B).

This outcome strongly suggests that molecule 58 exhibits a highly selective binding affinity for REG□ over the 20S core.

**Effects of compound 58 on REGγ-mediated peptide degradation by 20S proteasome.**

Inventors previously demonstrated that when REGγ binds to the 20S proteasome, it promotes the degradation of MHC-I antigenic epitopes. These findings were established through both *in vitro* degradation assays and T cell experiments. To investigate the impact of compound 58 on the degradation of the same peptide used in our earlier study, they utilized the same *in vitro* degradation assay. In this context, inventors employed a 52-amino acid peptide, KH-52, containing the SIINFEKL epitope at its core. The SIINFEKL epitope is a potent immunogenic component present in the ovalbumin protein, commonly employed in antigenic peptide presentation studies. In their experiment, KH-52 was subjected to *in vitro* incubation with human constitutive 20S proteasome (h20S) at 37°C for several hours. They gauged the rates of peptide bond hydrolysis by monitoring the generation of new amino groups through fluorescamine. KH-52, when incubated solely with h20S, exhibited consistent degradation over the entire incubation period (Figure 4A, depicted by the black dashed curve). Furthermore, as anticipated, the presence of REGγ slightly enhanced the rates of peptide bond hydrolysis (Figure 3A, comparing the solid black curve with the dashed black curve). Inventors also conducted the same experiment in the presence of compound 58 at different concentrations. Notably, compound 58 had no impact on the rate of peptide bond hydrolysis by h20S across the different concentrations tested (Figure 4A, grey dash curves compared to the black dash curve). Conversely, when compound 58 was utilized at a concentration of 50 μM, it notably decelerated the rate of peptide bond hydrolysis in the 20S-REGy complex (Figure 4A, contrasting the solid black curve with the solid grey curve), as demonstrated in Figure 4B.

These results show a role for compound 58 on the REGg-20S complex in modulating peptide degradation, which further highlights its importance in the context of immune response regulation.

**Assessment of compound 58's IC50 values and differential responsiveness in mouse tumor cell lines.**

To assess the efficacy of compound 58 against tumor cell lines, inventors initially aimed to determine its IC50 values in both mouse and human cell lines. They utilized an MTT assay to ascertain these values for mouse MCA fibrosarcoma and human A375 melanoma cell lines. Specifically, they treated $2.5 \times 10^4$ MCA WT or MCA REGγ KO cells and $5 \times 10^4$ human A375 WT or A375 REGγ KO cells with varying concentrations of compound 58 for a 24-hour duration.

The MTT assay results revealed that following a 24-hour treatment, compound 58 reduced the cell viability as follows: an IC50 value of 39.09 μM for MCA WT, an IC50 value of 99.63 μM for MCA REGγ KO (Figure 5A), an IC50 value of 149.6 μM for A375 WT, and an IC50 value of 486.4 μM for A375 REGγ KO (Figure 5B). These results indicate that REGγ KO cell lines are less responsive to compound 58 compared to their WT counterparts.

**Compound 58 enhances antigen presentation and immune response in mouse tumor cells.**

Inventors previously reported a novel and unexpected role of the REGγ-20S complex in the degradation of MHC-I peptides in cancer. In order to elucidate the potential impact of compound 58 on the REGγ-20S complex's modulation of antigenic peptide degradation, they treated mouse MCA205 cells stably expressing the globin-SL8-intron construct with increasing concentrations of compound 58 for 24 hours. Employing flow cytometry and the 25-D1.16 monoclonal antibody, which specifically recognizes Kb-SIINFEKL complexes on the cell surface, they demonstrated that treatment with 25μM of compound 58 led to a significant increase in the presentation of the SIINFEKL peptide compared to untreated cells. This result underscores the ability of compound 58 to counteract the inhibitory effect of REGγ on antigenic peptide degradation by the 20S core (see Figure 6A).

Additionally, they explored the impact of compound 58 on the expression of MHC-I on the surface of MCA205 cells stably expressing the globin-SL8-intron construct. After treating these cells with varying doses of compound 58 for 24 hours, they assessed the levels of murine MHC-I $H2\text{-}K^b$ and $H2\text{-}D^b$ using flow cytometry. The percentage of cells labeled positively for $H2\text{-}K^b$ and $H2\text{-}D^b$ in the treated conditions was compared to that in the DMSO control condition. Notably, they observed a dose-dependent increase in the expression of $H2\text{-}K^b$ and $H2\text{-}D^b$ molecules on the surface of MCA205 cells following treatment with compound 58 *in vitro* from 5μm to 25 μM (Figures 6B and 6C).

Furthermore, inventors investigated whether compound 58 had the capability to alter PD-L1 expression on the cell surface of treated tumor cells. To examine this, they exposed MCA205 cells stably expressing the globin-SL8-intron construct to varying concentrations of compound 58 for 24 hours and subsequently assessed PD-L1 expression levels via flow cytometry. Their findings demonstrated that compound 58 significantly increased PD-L1 expression on the surface of

MCA205 cells in a dose-dependent manner *in vitro* (Figure 6D).

Collectively, these results underscore the ability of compound 58 to enhance the expression of MHC-I molecules on the surface of mouse tumor cells, thereby promoting increased presentation of peptide/MHC-I Kb complexes. This effect is particularly noteworthy as inhibition of the REGD-20S complex by the compound 58 amplifies the pool of antigenic peptides and augments the expression of MHC-I molecules, thereby facilitating the expression of SIINFEKL/MHC-1 Kb complexes on the cell surface.

**Compound 58 modulates OT-1 CD8+ T cell activation and effector functions**

Inventors previously demonstrated that compound 58 possesses the ability to inhibit the REGD-20S complex's capacity to destroy antigenic epitopes by binding to the REGγ regulator. Additionally, they established that compound 58 can enhance the expression of MHC-I molecules on the cell surface, consequently increasing the presentation of SIINFEKL/MHC-1 Kb complexes. Inventors hypothesize that this specific enhancement of this complex may stimulate the proliferation of specific CD8+ T cells. To investigate this, they employed OT-1 cells, which are TCR transgenic CD8+ T lymphocytes from OT-1 mice targeting OVA SL8 epitopes presented on murine MHC class I molecules (Kb).

In their experiments, OT-1 cells were co-incubated with MCA205 cells expressing the globin-SL8-intron construct. To assess the production of antigenic peptide substrates, they measured OT-1 cell proliferation using FACS analysis. They analyzed the expression levels of early activation markers, CD69 and CD25, which are critical for the proliferation of activated T cells. For that purpose, MCA205 cells with the globin-SL8-intron construct were exposed to varying concentrations of compound 58 for 24 hours, followed by co-incubation with OT-1 cells. Notably, treatment with compound 58 led to an increased frequency of CD69+ CD25+ OT-1 T cells compared to untreated cells, confirming the activation of OT-1 cells upon exposure to compound 58.

Furthermore, inventors sought to characterize the activated OT-1 cell subset induced by this treatment. To do so, they examined the expression of CD44 and CD62L (L-selectin) markers, which delineate two major subsets of OT-1 CD8+ T cells following co-culture with MCA cells treated with compound 58: effector/memory ($CD44^{high}/CD62L^{low}$) and central memory ($CD44^{high}/CD62L^{high}$) CD8+ T cell subsets. The frequency of $CD44^{high}/CD62L^{low}$ effector/memory cells exhibited a dose-dependent increase, whereas the frequency of central memory $CD44^{high}/CD62L^{high}$ remained unchanged, regardless of treatment with compound 58.

Inventors then evaluated the effector function of OT-1 cells post-co-culture with MCA cells treated with compound 58 by examining the expression of perforin. Notably, OT-1 cells exhibited enhanced cytotoxic activity, as evidenced by increased perforin expression.

In summary, inventors' findings support the notion that compound 58 activates and enhances the effector functions of OT-1 CD8+ T cells, particularly the effector/memory subset, and may have implications for boosting immune responses, in particular cancer immune response.

## CONCLUSION

Here, inventors demonstrate the discovery of a promising molecule through an extensive high-throughput screening process involving over 2100 compounds from NIH compound libraries. This molecule has shown the capability to inhibit the influence of REGγ on the 20S proteasome concerning antigen presentation. Notably, it directly binds to the REGγ complex. Additionally, compound 58 has been found to significantly enhance the expression of MHC-I molecules on the surface of mouse tumor cells. Consequently, this augmentation facilitates an increased presentation of peptide/MHC-I Kb complexes, thereby activating and reinforcing the effector functions of OT-1 CD8+ T cells, with a specific emphasis on the effector/memory subset.

## Sequence listing

SEQ ID NO: 1 (epitope contained within an intron of the human β-globin gene): SIINFEKL

SEQ ID NO: 2 (52 amino acid peptide KH-52):
KVNVDEVGGEALGRLVSRLQDRSIINFEKLFKETNRNWACGDREDSWVSDRH

## REFERENCES

- Apcher, S., B. Vojtesek, and R. Fahraeus, In search of the cell biology for self- versus non-self-recognition. Curr Opin Immunol, 2023. 83: p. 102334.
- Apcher, S., et al., mRNA translation from an antigen presentation perspective: A tribute to the works of Nilabh Shastri. Mol Immunol, 2022. 141: p. 305-308.
- Apcher, S., et al., Translation of pre-spliced RNAs in the nuclear compartment generates peptides for the MHC class I pathway. Proc Natl Acad Sci USA, 2013. 110(44): p. 17951-6.
- Apcher, S., et al., Major source of antigenic peptides for the MHC class I pathway is produced during the pioneer round of mRNA translation. Proc Natl Acad Sci USA, 2011. 108(28): p. 11572-7.
- Boulpicante, M., et al., Tumors escape immunosurveillance by overexpressing the proteasome activator PSME3. Oncoimmunology, 2020. 9(1): p. 1761205.
- Cascio, P., PA28alphabeta: the enigmatic magic ring of the proteasome? Biomolecules, 2014. 4(2): p. 566-84.
- Chai, F., et al., High expression of REGgamma is associated with metastasis and poor prognosis of patients with breast cancer. Int J Clin Exp Pathol, 2014. 7(11): p. 7834-43.
- Chen, D., et al., The expression and clinical significance of PA28 gamma in colorectal cancer. J Investig Med, 2013. 61(8): p. 1192-6.
- Chen, X., et al., Ubiquitin-independent degradation of cell-cycle inhibitors by the REGgamma proteasome. Mol Cell, 2007. 26(6): p. 843-52.
- Duvallet, E., et al., Exosome-driven transfer of tumor-associated Pioneer Translation Products (TA-PTPs) for the MHC class I cross-presentation pathway. Oncoimmunology, 2016. 5(9): p. e1198865.
- Funderburk, K.E., J. Kang, and H.J. Li, Regulation of Life & Death by REGgamma. Cells, 2022. 11(15).
- Garin, J., Diez, R., Kieffer, S., Dermine, J. F., Duclos, S., Gagnon, E., Sadoul, R., Rondeau, C. and Desjardins, M. The phagosome proteome: insight into phagosome functions. J Cell Biol 152, 165-80., 2001.
- Huang, L., et al., Proteasome activators, PA28gamma and PA200, play indispensable roles in male fertility. Sci Rep, 2016. 6: p. 23171.
- Jonik-Nowak, B., et al., PIP30/PAM192A is a novel regulator of the nuclear proteasome activator PA28gamma. Proc Natl Acad Sci USA, 2018. 115(28): p. E6477-E6486.
- Khare G., et al., Identification of inhibitors against Mycobacterium tuberculosis thiamin phosphate synthase, an important target for the development of anti-TB drugs. PLoS One. 2011;6(7):e22441.
- Kontos, C.K., Surrogate Prognostic Biomarkers in OSCC: The Paradigm of PA28gamma Overexpression. EBio-Medicine, 2015. 2(8): p. 784-5.
- Le Feuvre A.Y., et al., High yield bacterial expression and purification of active recombinant PA28$\alpha\beta$ complex. Protein Expr. Purif. 2009;64:219-224.
- Lei, K., et al., PA28gamma, an Accomplice to Malignant Cancer. Front Oncol, 2020. 10: p. 584778.
- Lesne, J., et al., Conformational maps of human 20S proteasomes reveal PA28- and immuno-dependent inter-ring crosstalks. Nat Commun, 2020. 11(1): p. 6140.
- Li, J. and M. Rechsteiner, Molecular dissection of the 11S REG (PA28) proteasome activators. Biochimie, 2001. 83(3-4): p. 373-83.
- Li, X., et al., Ubiquitin- and ATP-independent proteolytic turnover of p21 by the REGgamma-proteasome pathway. Mol Cell, 2007. 26(6): p. 831-42.
- Li, X., et al., The SRC-3/AIB1 coactivator is degraded in a ubiquitin- and ATP-independent manner by the REGgamma proteasome. Cell, 2006. 124(2): p. 381-92.
- Realini, C., et al., Characterization of recombinant REGalpha, REGbeta, and REGgamma proteasome activators. J Biol Chem, 1997. 272(41): p. 25483-92.
- Rechsteiner, M., C. Realini, and V. Ustrell, The proteasome activator 11 S REG (PA28) and class I antigen presentation. Biochem J, 2000. 345 Pt 1: p. 1-15.
- Shastri, N., et al., All the peptides that fit: the beginning, the middle, and the end of the MHC class I antigen-processing pathway. Immunol Rev, 2005. 207: p. 31-41.
- Song, W., et al., Silencing PSME3 induces colorectal cancer radiosensitivity by downregulating the expression of cyclin B1 and CKD1. Exp Biol Med (Maywood), 2019. 244(16): p. 1409-1418.
- Sroka, E.M., et al., Major histocompatibility class I antigenic peptides derived from translation of pre-mRNAs generate immune tolerance. Proc Natl Acad Sci USA, 2023. 120(7): p. e2208509120.
- Thery, C., Boussac, M., Veron, P., Ricciardi-Castagnoli, P., Raposo, G., Garin, J. and Amigorena, S. Proteomic analysis of dendritic cell-derived exosomes: a secreted subcellular compartment distinct from apoptotic vesicles. J Immunol 166, 7309-18., 2001.
- Thery, C., Regnault, A., Garin, J., Wolfers, J., Zitvogel, L., Ricciardi-Castagnoli, P., Raposo, G. and Amigorena, S. Molecular characterization of dendritic cell-derived exosomes. Selective accumulation of the heat shock protein hsc73. J Cell Biol 147, 599-610, 1999.
- Thery C., Zitvogel L. and Amigorena S. Exosomes: Composition, Biogenesis and Function. Nature review 2 (2002)

569.
- Thomas, T.A. and D.M. Smith, Proteasome activator 28gamma (PA28gamma) allosterically activates trypsin-like proteolysis by binding to the alpha-ring of the 20S proteasome. J Biol Chem, 2022. 298(8): p. 102140.
- Vigneron, N. and B.J. Van den Eynde, Proteasome subtypes and regulators in the processing of antigenic peptides presented by class I molecules of the major histocompatibility complex. Biomolecules, 2014. 4(4): p. 994-1025.
- Wilk, S., W.E. Chen, and R.P. Magnusson, Properties of the nuclear proteasome activator PA28gamma (REGgamma). Arch Biochem Biophys, 2000. 383(2): p. 265-71.
- Yewdell, J.W., L.C. Anton, and J.R. Bennink, Defective ribosomal products (DRiPs): a major source of antigenic peptides for MHC class I molecules? J Immunol, 1996. 157(5): p. 1823-6.
- Wolfers, J., Lozier, A., Raposo, G., Regnault, A., Thery, C., Masurier, C., Flament, C., Pouzieux, S., Faure, F., Tursz, T., Angevin, E., Amigorena, S. and Zitvogel, L. Tumor-derived exosomes are a source of shared tumor rejection antigens for CTL cross-priming. Nat Med 7, 297-303., 2001.

Zitvogel, L., Regnault, A., Lozier, A., Wolfers, J., Flament, C., Tenza, D., Ricciardi-Castagnoli, P., Raposo, G. and Amigorena, S. Eradication of established murine tumors using a novel cell-free vaccine: dendritic cell-derived exosomes. Nat Med 4, 594-600, 1998 .

## Claims

**1.** A Compound of formula (I):

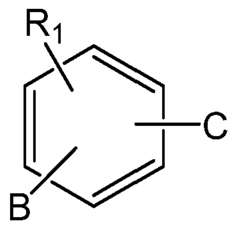

(I)

wherein

- $R_1$ is H or a $C_1$-$C_6$ alkyl
- B is of formula (b):

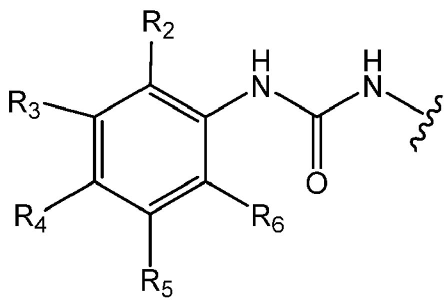

(b)

and
- C is of formula (c):

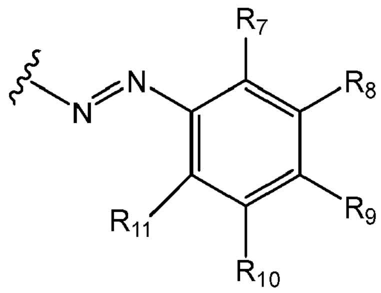

(c)

wherein:
each $R_2$, $R_3$, $R_4$, $R_5$, $R_6$, $R_7$, $R_8$, $R_9$, $R_{10}$, and $R_{11}$ is independently selected from H, halogen atom, -OH, a $C_1$-$C_6$ alkyl, $C_1$-$C_6$ alkoxy, $C_1$-$C_6$ halogenoalkyl, 4- to 6-membered heterocycle preferably 4- to 6-membered heterocycloalkyl group, $C_1$-$C_6$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or a $C_1$-$C_6$ alkyl,
or a pharmaceutically acceptable salt and/or solvate thereof, for use for treating an immunodeficient subject.

**2.** The Compound for use according to claim 1, wherein

- $R_1$ is H or $-CH_3$ and/or
- each $R_2$, $R_3$, $R_4$, $R_5$, $R_6$, $R_7$, $R_8$, $R_9$, $R_{10}$, and $R_{11}$ is independently selected from H, halogen atom preferably -Cl, -Br or -F, $C_1$-$C_3$ alkyl, OH, $C_1$-$C_3$ alkoxy, $C_1$-$C_3$ halogenoalkyl such as $CF_3$, -COOH, $-OPO(OH)_2$, $B(OH)_2$, $-SO_3H$, $-NH_2$ and $-NO_2$.

**3.** The compound for use according to claim 1, wherein the compound is of formula (Ia):

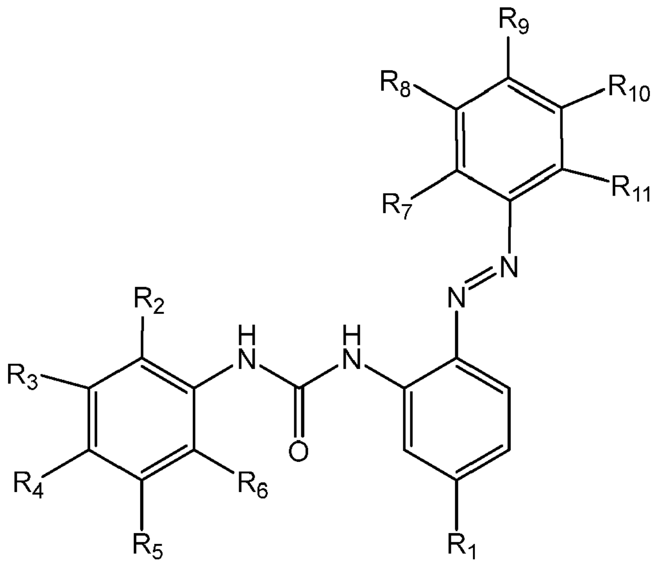

(Ia)

wherein $R_1$, $R_2$, $R_3$, $R_4$, $R_5$, $R_6$, $R_7$, $R_8$, $R_9$, $R_{10}$, and $R_{11}$ being as defined in claim 1,
or a pharmaceutically acceptable salt and/or solvate thereof.

**4.** The compound for use according to claim 3 wherein

- $R_1$ is H or a $C_1$-$C_3$ alkyl, preferably $-CH_3$,
- $R_8$, $R_8$, $R_{10}$, and $R_{11}$ are H, and
- each $R_2$, $R_3$, $R_4$, $R_6$, $R_7$, and $R_9$, is independently selected from H, halogen atom, -OH, a $C_1$-$C_3$ alkyl, $C_1$-$C_3$ alkoxy, $C_1$-$C_3$ halogenoalkyl, a $C_1$-$C_3$ hydroxyalkyl, $-NO_2$, $-B(OR_{12})_2$, $-OP(O)(OR_{12})_2$, $-N(R_{12})_2$, $-CONHR_{12}$, $-SO_3R_{12}$ and $-COOR_{12}$ with $R_{12}$ is H or a $C_1$-$C_3$ alkyl, preferably $R_{12}$ is H.

**5.** The compound for use according to claim 4, which is

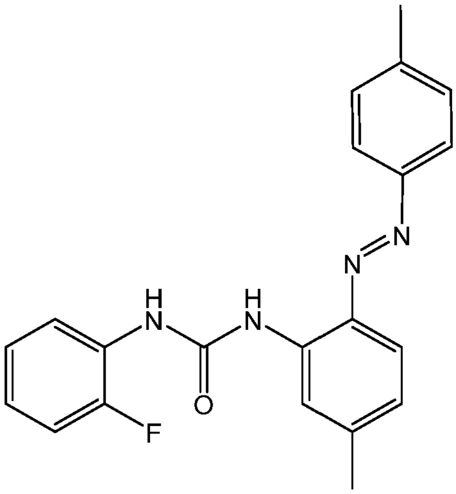

(compound 58)

or a pharmaceutically acceptable salt and/or solvate thereof.

**6.** A composition comprising a compound as defined in any one of claims 1 to 5, and a pharmaceutically acceptable carrier.

**7.** A combination of (a) a compound as defined in any one of claims 1 to 5 and (b) a distinct therapeutic agent, preferably selected from an immunotherapeutic agent, an anti-cancer agent and a combination thereof, or a composition comprising the combination of (a) and (b) and a pharmaceutically acceptable carrier.

**8.** The combination or composition of claim 7, wherein the compound is

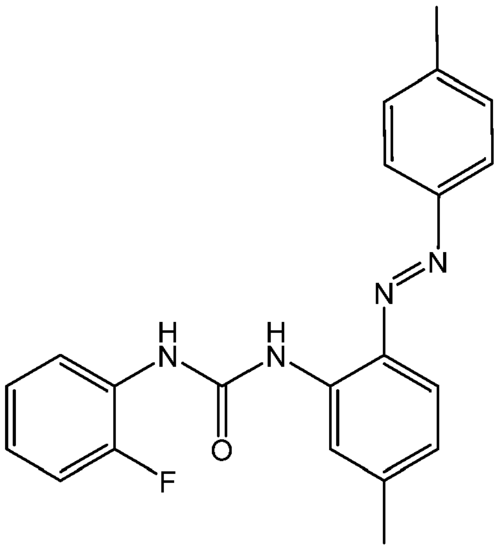

or a pharmaceutically acceptable salt and/or solvate thereof.

**9.** The combination or composition according to claim 7 or 8, wherein the distinct therapeutic agent (b) is an anticancer agent selected from an antimetabolic agent, an alkylating agent, a vinca-alkaloid, an intercalating agent, a tyrosine kinase inhibitor (TKI), an antibody or a fragment thereof, an antibodydrug conjugate ("ADC"), an anti-angiogenic agent, an immune check point inhibitor, a CAR-T cell, a toxoid, and an oncolytic virus, in particular an oncolytic virus-based immunotherapeutic agent.

**10.** The combination or composition according to anyone of claims 6 to 9, for use as a medicament.

**11.** The compound as defined in any one of claims 1 to 5, the composition as defined in claim 6, or the combination or

composition according to any one of claims 7 to 9, wherein said compound, combination or composition is for use for preventing or treating a disease selected from a cancer, an inflammation, and an infectious disease, preferably an infectious disease which is not induced by M. *tuberculosis.*

**12.** The combination or composition for use according to any one of claims 10 or 11, wherein (a) and (b) are formulated for simultaneous, concurrent or sequential administration by the same administration route or by different administration routes.

**13.** The compound, the composition or the combination or composition according to any one of claims 10 to 12, for use for treating a cancer in a subject, in particular a cancer wherein, compared to the healthy cells of the subject, the tumor cells of the subject overexpress REGg.

**14.** The compound, combination or composition for use according to claim 13, wherein the cancer is preferably a metastatic cancer or a cancer resistant and/or refractory to standard-of-care treatment, in particular to immunotherapy.

**15.** The combination or composition for use according to any one of claims 10 to 14, wherein the compound is present in the combination or composition at a dose of about 1 mg to about 200 mg, preferably about 1 mg to about 50 mg, more preferably about 10 mg.

**16.** A pharmaceutical kit for use in the prevention or treatment of a disease selected from a cancer, an inflammation, a neurodegenerative disease, and an infectious disease, preferably an infectious disease which is not induced by *M. tuberculosis,* wherein the kit comprises i) a compound as defined in any one of claims 1 to 5, in particular as defined in claim 5, and ii) an additional distinct therapeutic agent, in distinct containers.

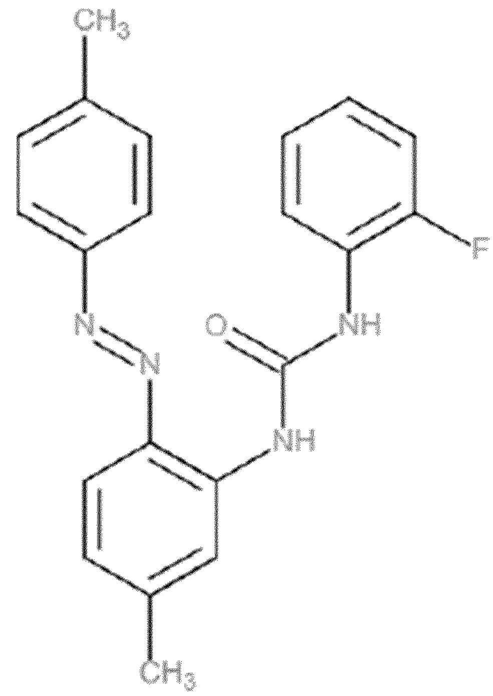

**FIGURE 1A**

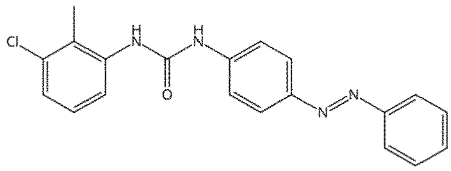

$C_{20}H_{17}ClN_4O$

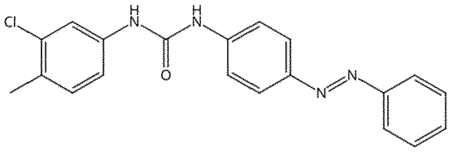

$C_{20}H_{17}ClN_4O$

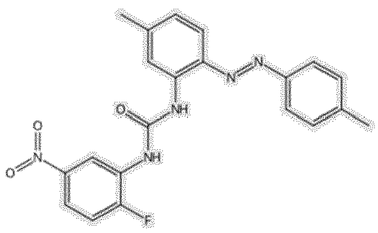

$C_{21}H_{18}FN_5O_3$

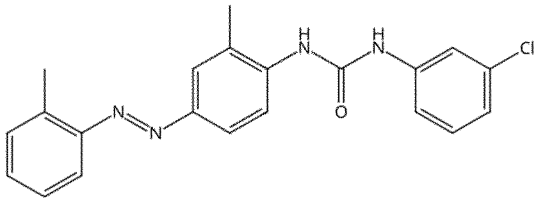

$C_{21}H_{19}ClN_4O$

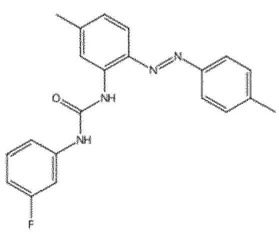

$C_{21}H_{19}FN_4O$

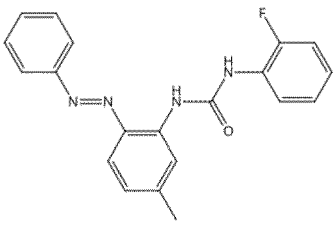

$C_{20}H_{17}FN_4O$

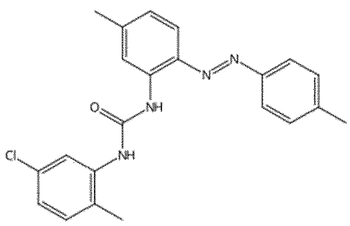

$C_{22}H_{21}ClN_4O$

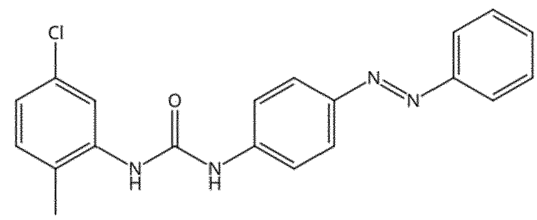

$C_{20}H_{17}ClN_4O$

**Figure 1B**

A

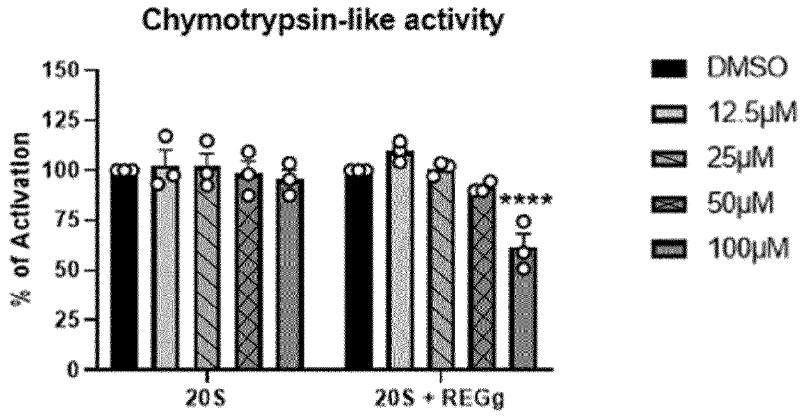
Chymotrypsin-like activity
% of Activation
150
125
100
75
50
25
0
****
20S
20S + REGg
DMSO
12.5µM
25µM
50µM
100µM

B

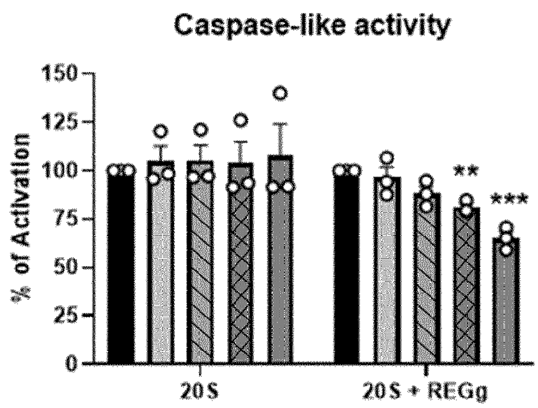
Caspase-like activity
% of Activation
150
125
100
75
50
25
0
**
***
20S
20S + REGg

C

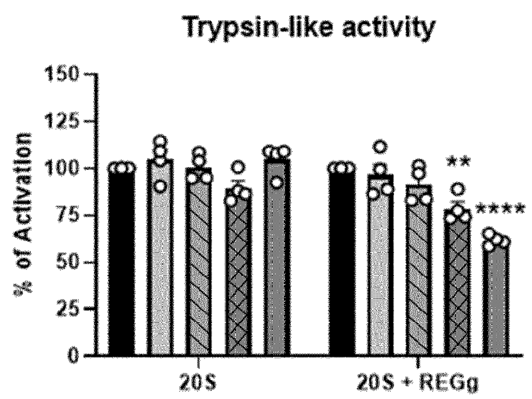
Trypsin-like activity
% of Activation
150
125
100
75
50
25
0
**
****
20S
20S + REGg

FIGURE 2

A

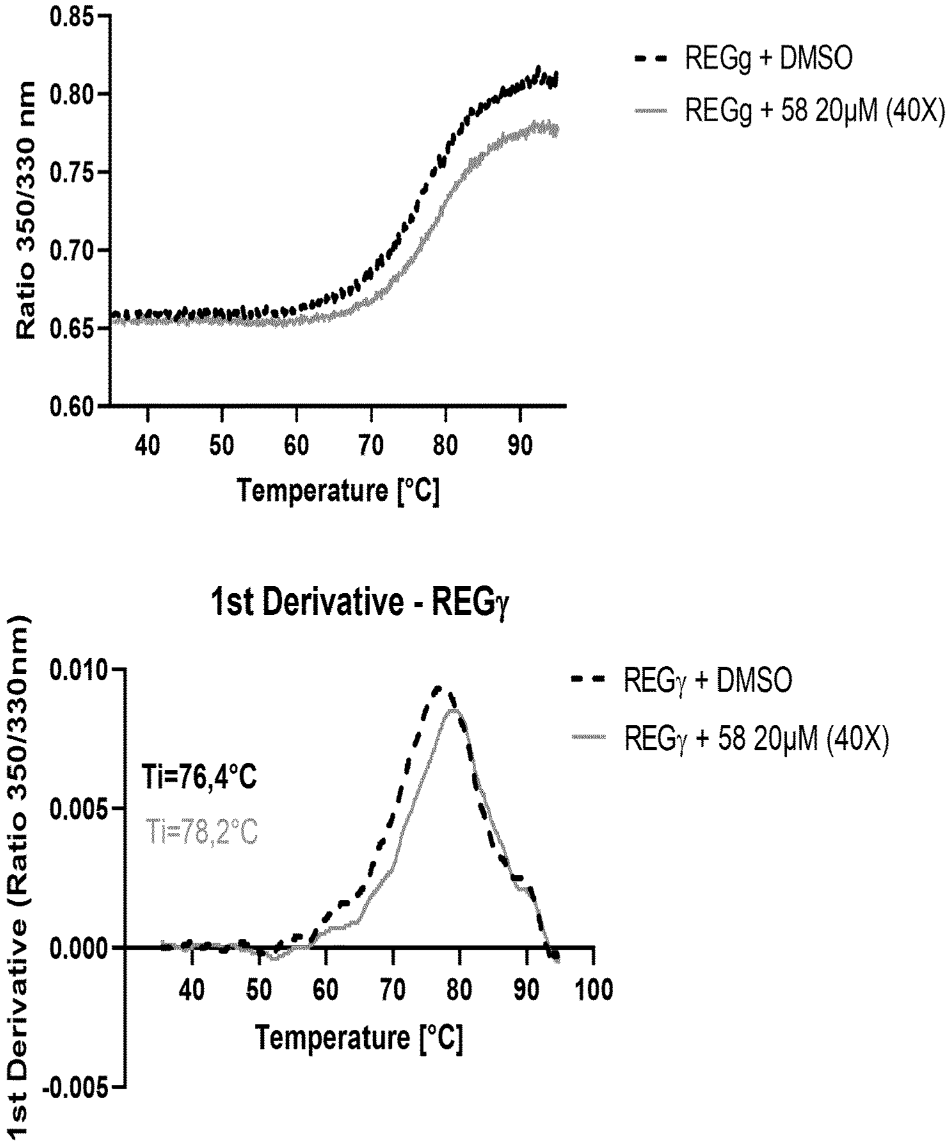
0.85
0.80
0.75
0.70
0.65
0.60
Ratio 350/330 nm
40
50
60
70
80
90
Temperature [°C]
REGg + DMSO
REGg + 58 20µM (40X)
1st Derivative - REGγ
0.010
0.005
0.000
-0.005
1st Derivative (Ratio 350/330nm)
Ti=76,4°C
Ti=78,2°C
40
50
60
70
80
90
100
Temperature [°C]
REGγ + DMSO
REGγ + 58 20µM (40X)

FIGURE 3

**B**

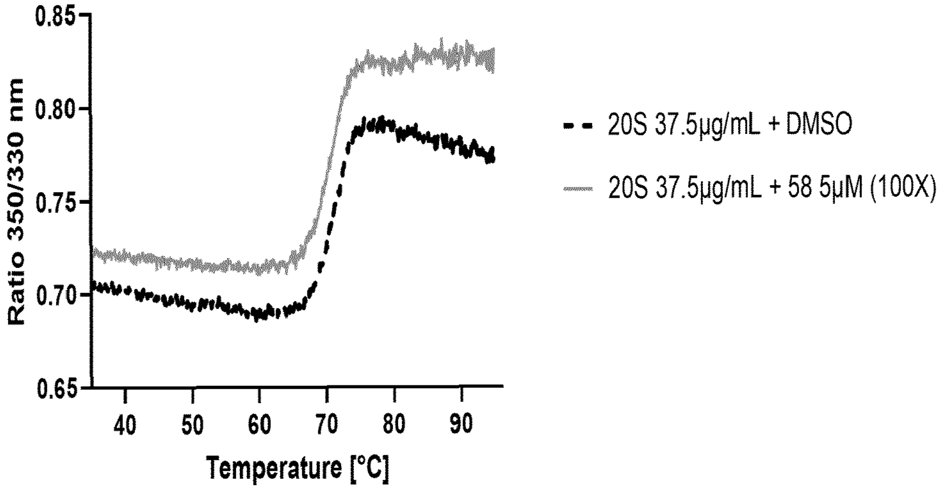


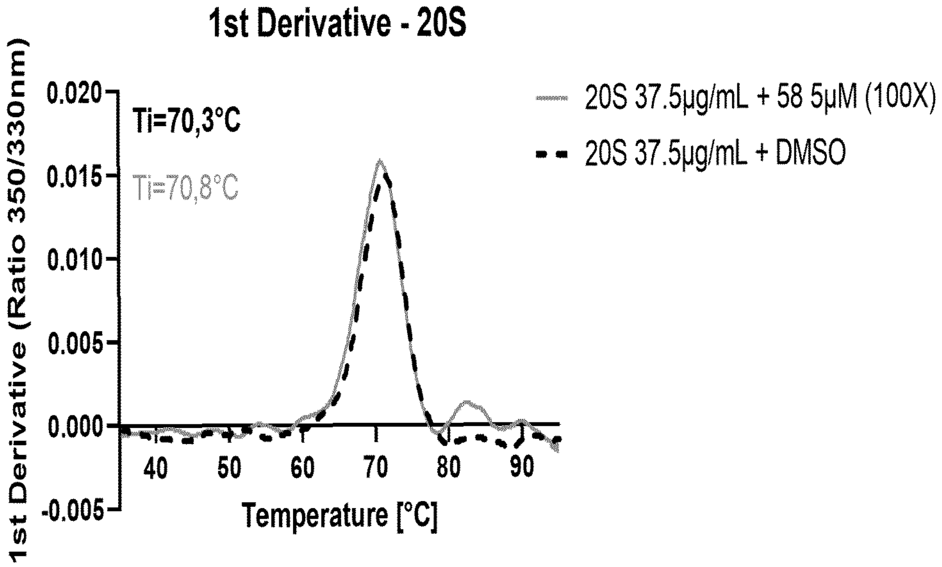


FIGURE 3 (Following)

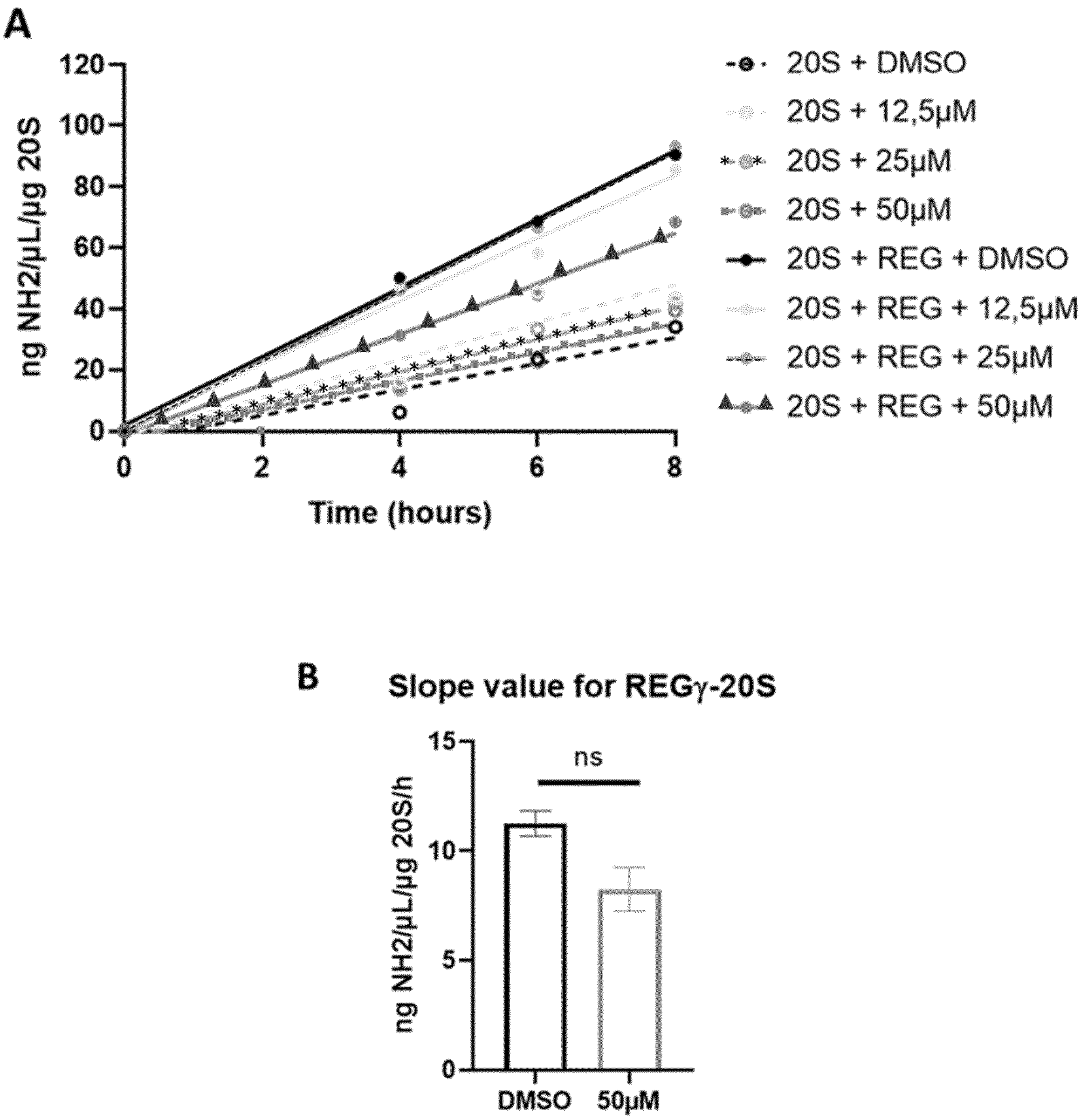
A
ng NH2/µL/µg 20S
Time (hours)
20S + DMSO
20S + 12,5µM
20S + 25µM
20S + 50µM
20S + REG + DMSO
20S + REG + 12,5µM
20S + REG + 25µM
20S + REG + 50µM
B
Slope value for REGγ-20S
ns
ng NH2/µL/µg 20S/h
DMSO
50µM

FIGURE 4

A

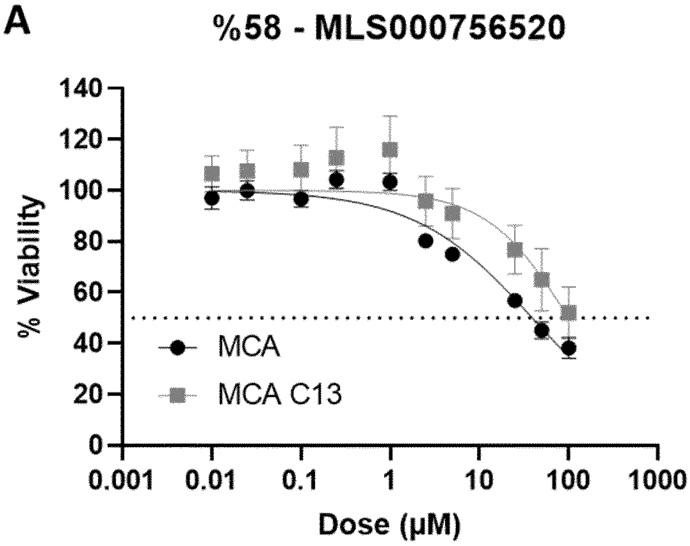


| | MCA | MCA c13 |
| --- | --- | --- |
| IC50 | 39,09µM | 99,63µM |

B

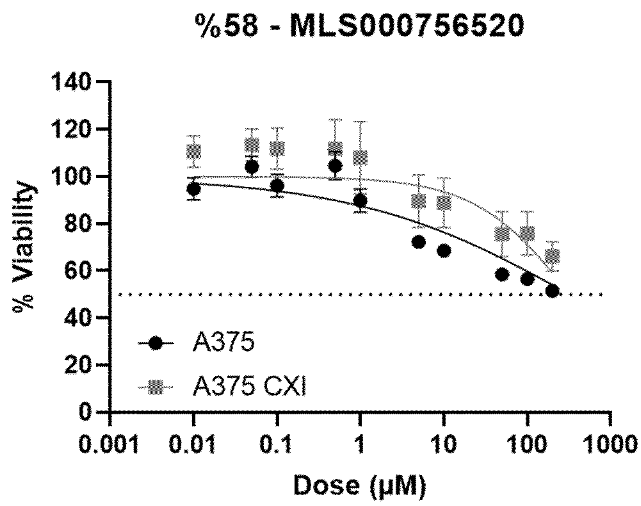


| | A375 | 1375 CXI |
| --- | --- | --- |
| IC50 | 149,6µM | 486,4µM |

FIGURE 5

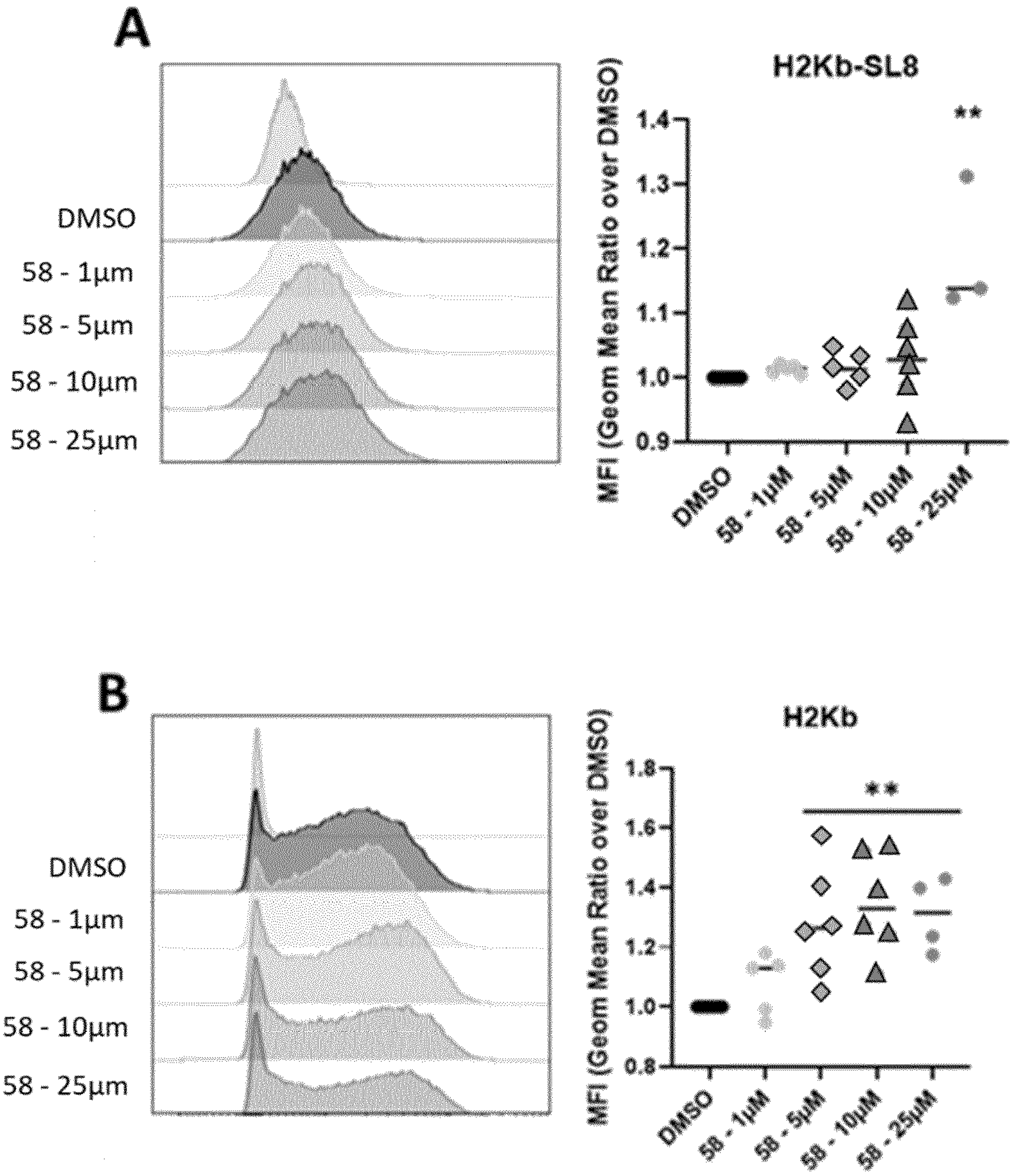
A
DMSO
58 - 1µm
58 - 5µm
58 - 10µm
58 - 25µm
H2Kb-SL8
MFI (Geom Mean Ratio over DMSO)
1.4
1.3
1.2
1.1
1.0
0.9
**
DMSO
58 - 1µM
58 - 5µM
58 - 10µM
58 - 25µM
B
DMSO
58 - 1µm
58 - 5µm
58 - 10µm
58 - 25µm
H2Kb
MFI (Geom Mean Ratio over DMSO)
1.8
1.6
1.4
1.2
1.0
0.8
**
DMSO
58 - 1µM
58 - 5µM
58 - 10µM
58 - 25µM

# FIGURE 6

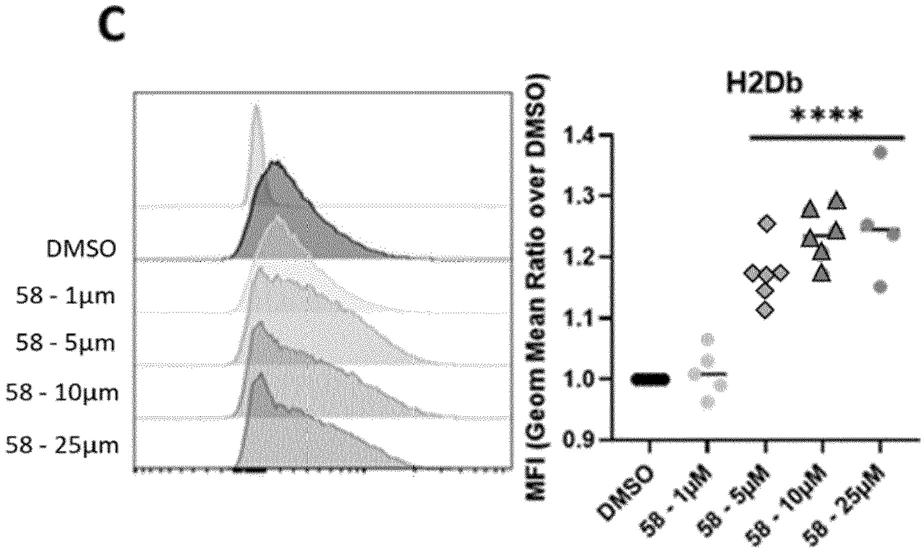


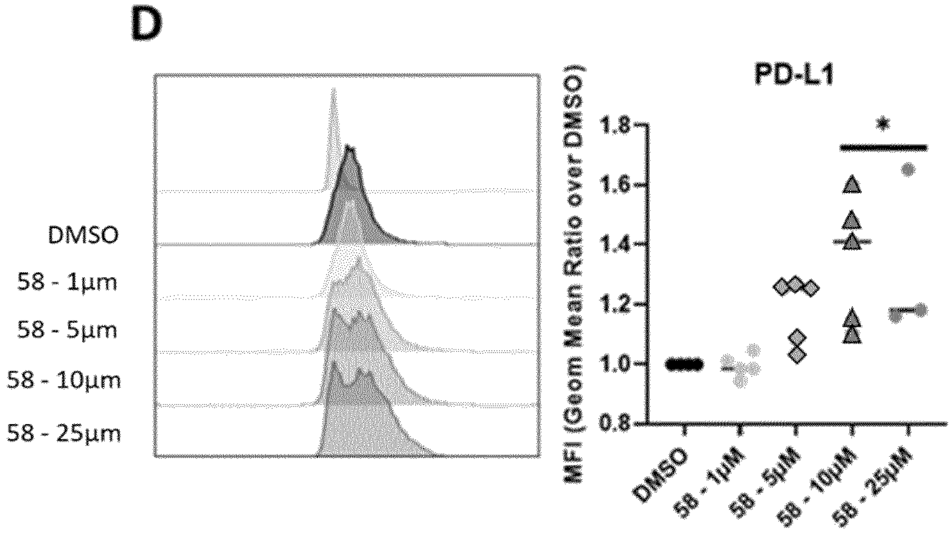


FIGURE 6 (Following)

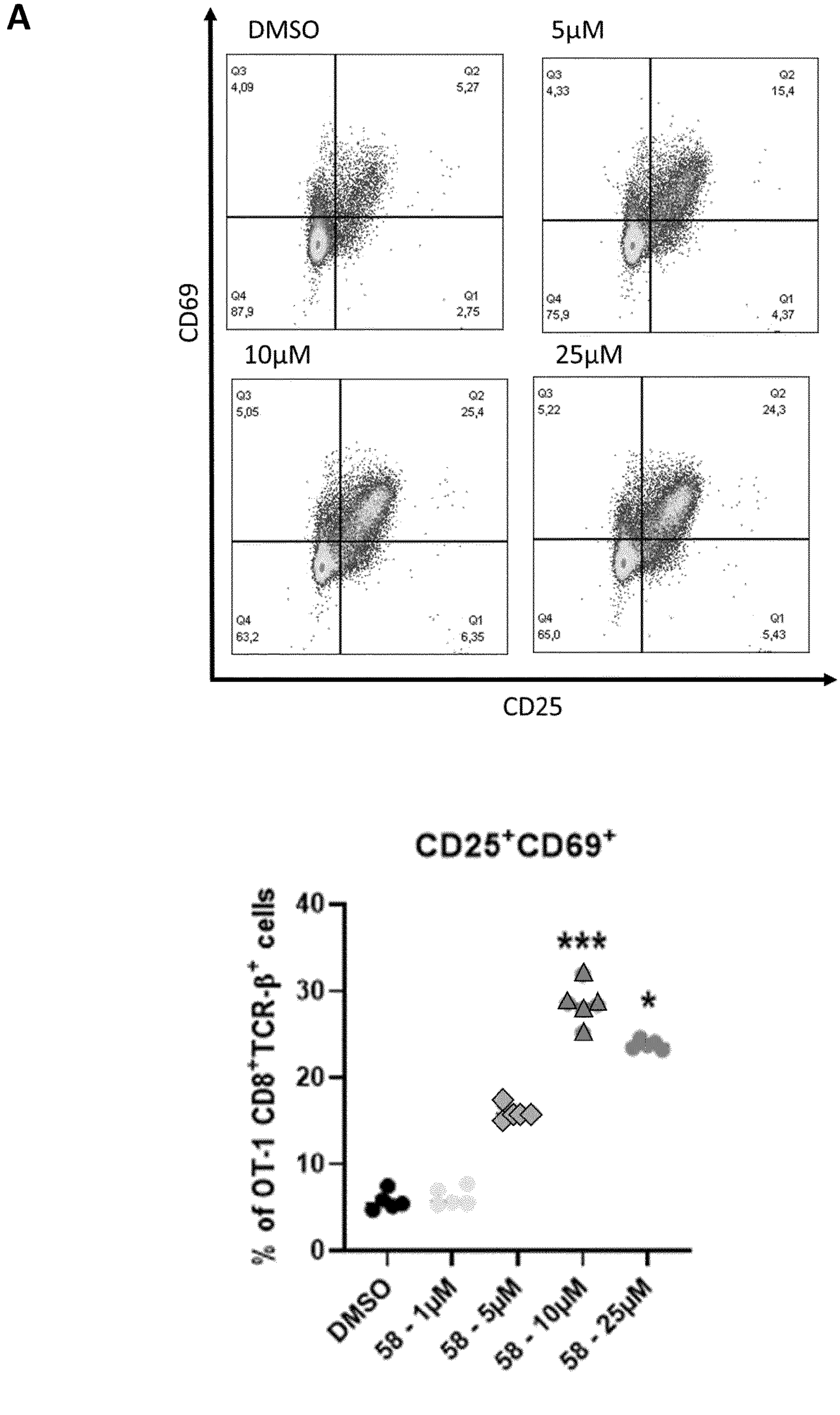
A
DMSO
5µM
10µM
25µM
Q3
4,09
Q2
5,27
Q4
87,9
Q1
2,75
Q3
4,33
Q2
15,4
Q4
75,9
Q1
4,37
Q3
5,05
Q2
25,4
Q4
63,2
Q1
6,35
Q3
5,22
Q2
24,3
Q4
65,0
Q1
5,43
CD69
CD25
CD25⁺CD69⁺
% of OT-1 CD8⁺TCR-β⁺ cells
40
30
20
10
0
***
*
DMSO
58 - 1µM
58 - 5µM
58 - 10µM
58 - 25µM

FIGURE 7A

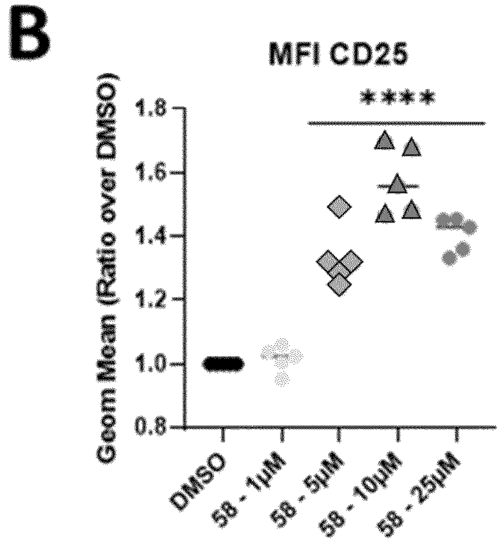


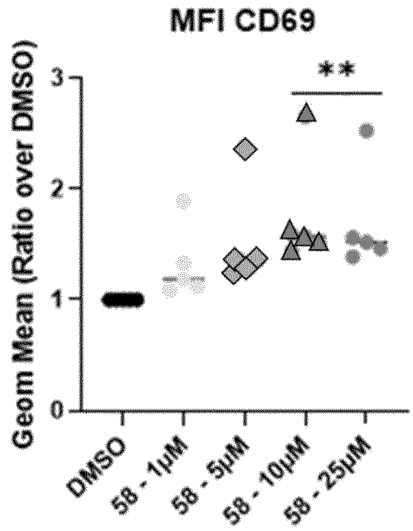


FIGURE 7B (Following)

C

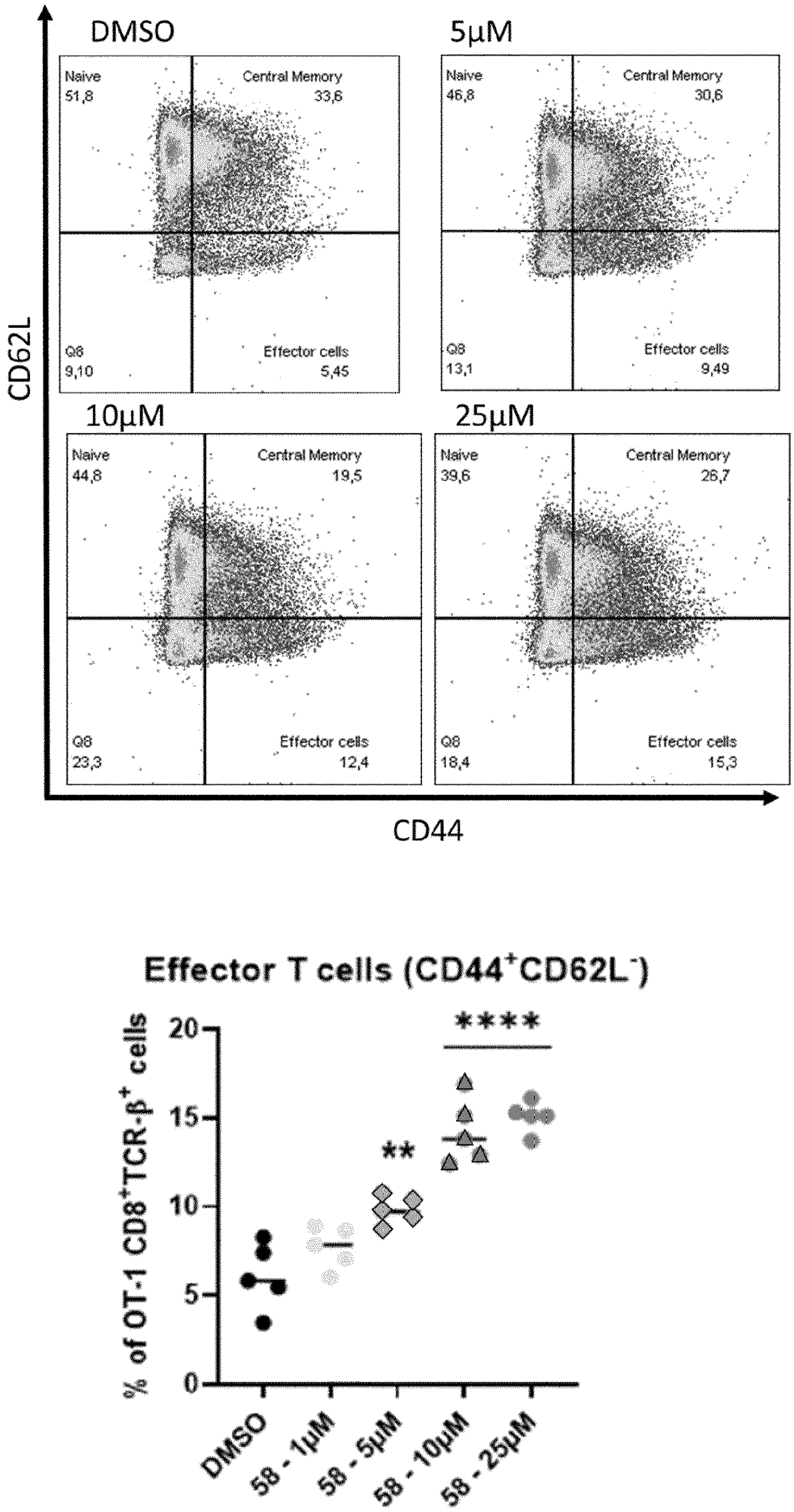


**FIGURE 7C (Following)**

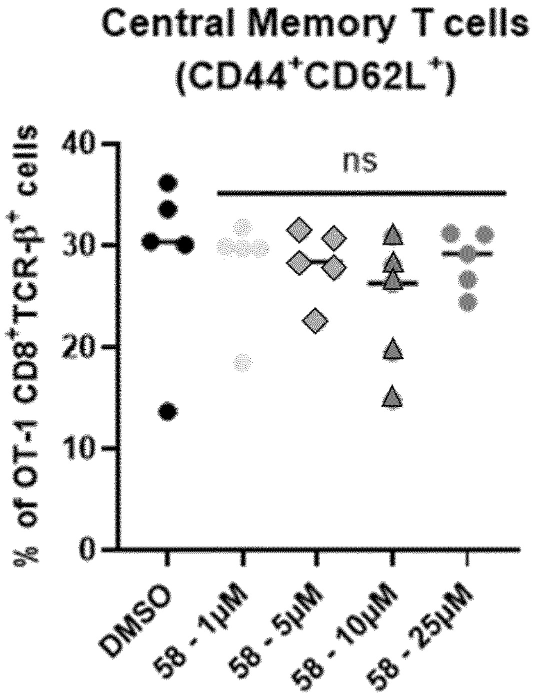


FIGURE 7C (Following)

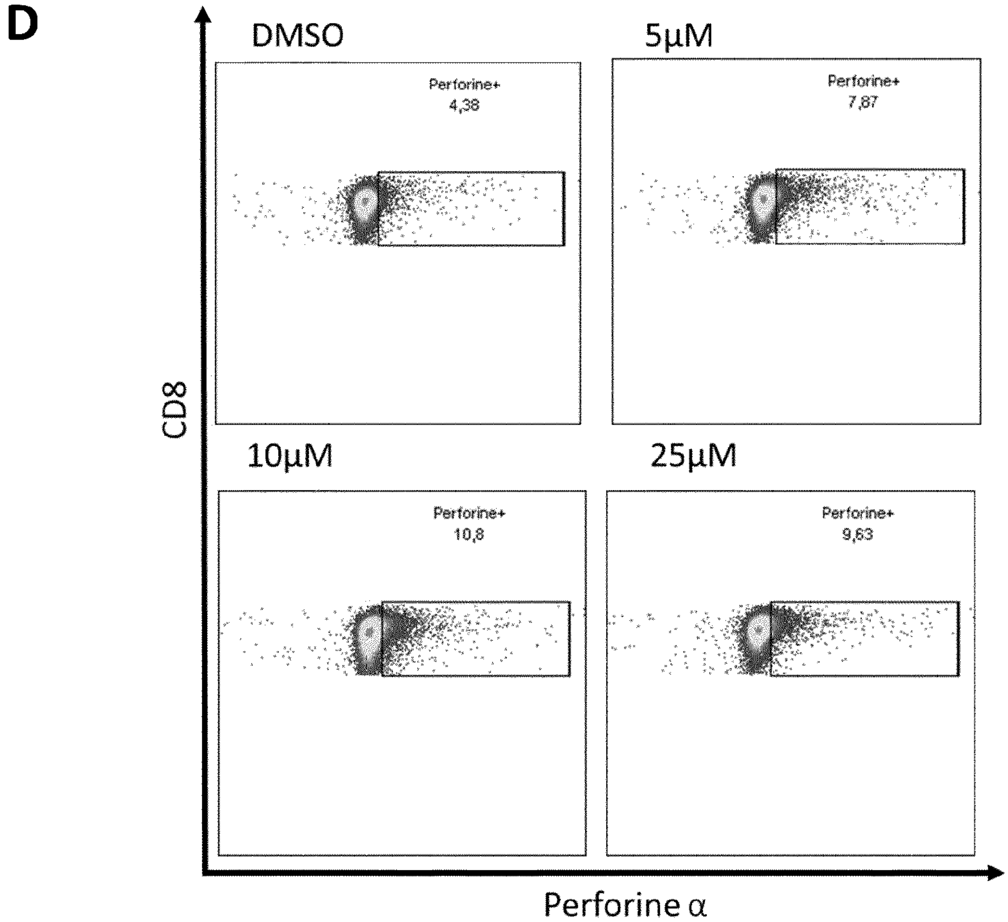


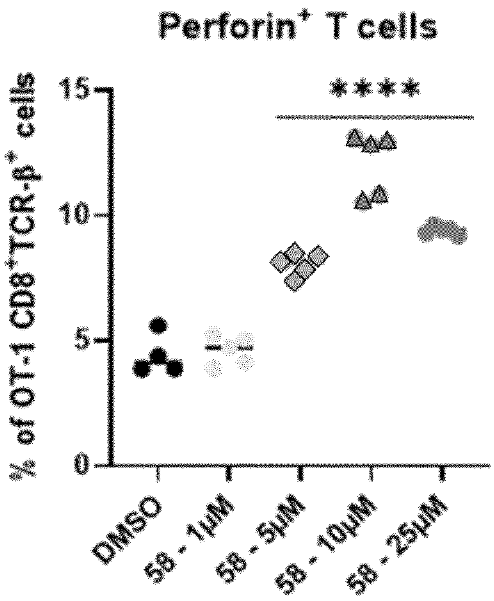


**FIGURE 7D (Following)**

Europäisches Patentamt
European Patent Office
Office européen des brevets

# EUROPEAN SEARCH REPORT

Application Number
EP 23 30 6787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 111 217 722 A (UNIV EAST CHINA SCIENCE & TECH) 2 June 2020 (2020-06-02)<br>* example 19 *<br>* table 1; compounds I18-I28, I30-I40, I85-I95, I252-I264, I269-I293 *<br>* claims *<br>----- | 1-16 | INV.<br>A61K31/17<br>A61K45/06<br>A61P29/00<br>A61P31/00<br>A61P31/12<br>A61P35/00<br>A61P35/04<br>A61P37/04 |
| X | WO 2010/138820 A2 (HARVARD COLLEGE [US]; AKTAS HUSEYIN [US] ET AL.)<br>2 December 2010 (2010-12-02)<br>* paragraphs [0009], [0086] *<br>* page 32; table 1; compound 1496 *<br>* claims 1, 3, 6, 8 *<br>----- | 1-16 | |
| X | KHARE GARIMA ET AL: "Identification of Inhibitors against Mycobacterium tuberculosis Thiamin Phosphate Synthase, an Important Target for the Development of Anti-TB Drugs",<br>PLOS ONE,<br>vol. 6, no. 7, 26 July 2011 (2011-07-26), page e22441, XP093142616,<br>US<br>ISSN: 1932-6203, DOI: 10.1371/journal.pone.0022441<br>Retrieved from the Internet:<br>URL:https://journals.plos.org/plosone/article/file?id=10.1371/journal.pone.0022441&t ype=printable><br>* abstract *<br>* page 3, right-hand column, line 5 - line 11 *<br>* page 5, right-hand column, line 35 - line 46 *<br>* page 9, right-hand column, line 15 - line 21 *<br>* figure 3 *<br>-/-- | 1-6 | TECHNICAL FIELDS SEARCHED (IPC)<br>A61K<br>A61P |

The present search report has been drawn up for all claims

1

| Place of search | Date of completion of the search | Examiner |
|---|---|---|
| Munich | 21 March 2024 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 30 6787

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| | & Khare Garima ET AL: "Supporting Information. Identification of Inhibitors against Mycobacterium tuberculosis Thiamin Phosphate Synthase, an Important Target for the Development of Anti-TB Drugs", PLOS ONE, 26 July 2011 (2011-07-26), XP093142656, Retrieved from the Internet: URL:https://storage.googleapis.com/plos-corpus-prod/10.1371/journal.pone.0022441/1/pone.0022441.s009.pdf?X-Goog-Algorithm=GOOG4-RSA-SHA256&X-Goog-Credential=wombat-sa@plos-prod.iam.gserviceaccount.com/20240319/auto/storage/goog4_request&X-Goog-Date=20240319T075551Z&X-Goog-Expires=86400&X-Goog-SignedHead [retrieved on 2024-03-19] * compound 30 * ----- | | |
| | | | TECHNICAL FIELDS SEARCHED (IPC) |

The present search report has been drawn up for all claims

1

| Place of search | Date of completion of the search | Examiner |
|---|---|---|
| Munich | 21 March 2024 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 23 30 6787

This annex lists the patent family members relating to the patent documents cited in the above-mentioned European search report. The members are as contained in the European Patent Office EDP file on
The European Patent Office is in no way liable for these particulars which are merely given for the purpose of information.

21-03-2024

| Patent document cited in search report | Publication date | Patent family member(s) | Publication date |
|---|---|---|---|
| CN 111217722 A | 02-06-2020 | NONE | |
| WO 2010138820 A2 | 02-12-2010 | AU 2010253820 A1 | 22-12-2011 |
| | | CA 2763589 A1 | 02-12-2010 |
| | | EP 2435040 A2 | 04-04-2012 |
| | | US 2012115915 A1 | 10-05-2012 |
| | | US 2016318857 A1 | 03-11-2016 |
| | | WO 2010138820 A2 | 02-12-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9903499 A
- WO 0044389 A
- WO 9705900 A
- WO 0028001 A
- WO 03016522 A
- WO 2017149118 A
- US 09780748 B


### Non-patent literature cited in the description


- **APCHER, S.** ; **B. VOJTESEK** ; **R. FAHRAEUS**. In search of the cell biology for self- versus non-self-recognition. *Curr Opin Immunol*, 2023, vol. 83, 102334
- **APCHER, S. et al.** mRNA translation from an antigen presentation perspective: A tribute to the works of Nilabh Shastri. *Mol Immunol*, 2022, vol. 141, 305-308
- **APCHER, S. et al.** Translation of pre-spliced RNAs in the nuclear compartment generates peptides for the MHC class I pathway.. *Proc Natl Acad Sci USA*, 2013, vol. 110 (44), 17951-6
- **APCHER, S. et al.** Major source of antigenic peptides for the MHC class I pathway is produced during the pioneer round of mRNA translation.. *Proc Natl Acad Sci USA*, 2011, vol. 108 (28), 11572-7
- **BOULPICANTE, M. et al.** Tumors escape immunosurveillance by overexpressing the proteasome activator PSME3. *Oncoimmunology*, 2020, vol. 9 (1), 1761205
- **CASCIO, P.** PA28alphabeta: the enigmatic magic ring of the proteasome?. *Biomolecules*, 2014, vol. 4 (2), 566-84
- **CHAI, F. et al.** High expression of REGgamma is associated with metastasis and poor prognosis of patients with breast cancer. *Int J Clin Exp Pathol*, 2014, vol. 7 (11), 7834-43
- **CHEN, D. et al.** The expression and clinical significance of PA28 gamma in colorectal cancer.. *J Investig Med*, 2013, vol. 61 (8), 1192-6
- **CHEN, X. et al.** Ubiquitin-independent degradation of cell-cycle inhibitors by the REGgamma proteasome. *Mol Cell*, 2007, vol. 26 (6), 843-52
- **DUVALLET, E. et al.** Exosome-driven transfer of tumor-associated Pioneer Translation Products (TA-PTPs) for the MHC class I cross-presentation pathway. *Oncoimmunology*, 2016, vol. 5 (9), e1198865
- **FUNDERBURK, K.E.** ; **J. KANG** ; **H.J. LI**. Regulation of Life & Death by REGgamma.. *Cells*, 2022, vol. 11 (15)
- **GARIN, J.** ; **DIEZ, R.** ; **KIEFFER, S.** ; **DERMINE, J. F.** ; **DUCLOS, S.** ; **GAGNON, E.** ; **SADOUL, R** ; **RONDEAU, C.** ; **DESJARDINS, M.** The phagosome proteome: insight into phagosome functions.. *J Cell Biol*, 2001, vol. 152, 165-80
- **HUANG, L. et al.** Proteasome activators, PA28gamma and PA200, play indispensable roles in male fertility. *Sci Rep*, 2016, vol. 6, 23171
- **JONIK-NOWAK, B. et al.** PIP30/PAM192A is a novel regulator of the nuclear proteasome activator PA28-gamma.. *Proc Natl Acad Sci USA*, 2018, vol. 115 (28), E6477-E6486
- **KHARE G. et al.** Identification of inhibitors against Mycobacterium tuberculosis thiamin phosphate synthase, an important target for the development of anti-TB drugs. *PLoS One.*, 2011, vol. 6 (7), e22441
- **KONTOS, C.K.** Surrogate Prognostic Biomarkers in OSCC: The Paradigm of PA28gamma Overexpression. *EBioMedicine*, 2015, vol. 2 (8), 784-5
- **LE FEUVRE A.Y. et al.** High yield bacterial expression and purification of active recombinant PA28$\alpha\beta$ complex. *Protein Expr. Purif.*, 2009, vol. 64, 219-224
- **LEI, K. et al.** PA28gamma, an Accomplice to Malignant Cancer. *Front Oncol*, 2020, vol. 10, 584778
- **LESNE, J. et al.** Conformational maps of human 20S proteasomes reveal PA28- and immuno-dependent inter-ring crosstalks.. *Nat Commun*, 2020, vol. 11 (1), 6140
- **LI, J.** ; **M. RECHSTEINER**. Molecular dissection of the 11S REG (PA28) proteasome activators. *Biochimie*, 2001, vol. 83 (3-4), 373-83
- **LI, X. et al.** Ubiquitin- and ATP-independent proteolytic turnover of p21 by the REGgamma-proteasome pathway. *Mol Cell*, 2007, vol. 26 (6), 831-42
- **LI, X. et al.** The SRC-3/AIB1 coactivator is degraded in a ubiquitin- and ATP-independent manner by the REGgamma proteasome.. *Cell*, 2006, vol. 124 (2), 381-92

- **REALINI, C. et al.** Characterization of recombinant REGalpha, REGbeta, and REGgamma proteasome activators.. *J Biol Chem*, 1997, vol. 272 (41), 25483-92
- **RECHSTEINER, M.** ; **C. REALINI** ; **V. USTRELL**. The proteasome activator 11 S REG (PA28) and class I antigen presentation.. *Biochem J*, 2000, vol. 345, 1-15
- **SHASTRI, N. et al.** All the peptides that fit: the beginning, the middle, and the end of the MHC class I antigen-processing pathway. *Immunol Rev*, 2005, vol. 207, 31-41
- **SONG, W. et al.** Silencing PSME3 induces colorectal cancer radiosensitivity by downregulating the expression of cyclin B1 and CKD1. *Exp Biol Med (Maywood)*, 2019, vol. 244 (16), 1409-1418
- **SROKA, E.M. et al.** Major histocompatibility class I antigenic peptides derived from translation of pre-mRNAs generate immune tolerance.. *Proc Natl Acad Sci USA*, 2023, vol. 120 (7), e2208509120
- **THERY, C.** ; **BOUSSAC, M.** ; **VERON, P.** ; **RICCIARDI-CASTAGNOLI, P.** ; **RAPOSO, G.** ; **GARIN, J.** ; **AMIGORENA, S**. Proteomic analysis of dendritic cell-derived exosomes: a secreted subcellular compartment distinct from apoptotic vesicles.. *J Immunol*, 2001, vol. 166, 7309-18
- **THERY, C.** ; **REGNAULT, A** ; **GARIN, J.** ; **WOLFERS, J.** ; **ZITVOGEL, L.** ; **RICCIARDI-CASTAGNOLI, P.** ; **RAPOSO, G.** ; **AMIGORENA, S**. Molecular characterization of dendritic cell-derived exosomes. Selective accumulation of the heat shock protein hsc73.. *J Cell Biol*, 1999, vol. 147, 599-610
- **THERY C.** ; **ZITVOGEL L.** ; **AMIGORENA S**. Exosomes: Composition, Biogenesis and Function.. *Nature review*, 2002, vol. 2, 569
- **THOMAS, T.A.** ; **D.M. SMITH**. Proteasome activator 28gamma (PA28gamma) allosterically activates trypsin-like proteolysis by binding to the alpha-ring of the 20S proteasome.. *J Biol Chem*, 2022, vol. 298 (8), 102140
- **VIGNERON, N.** ; **B.J. VAN DEN EYNDE**. Proteasome subtypes and regulators in the processing of antigenic peptides presented by class I molecules of the major histocompatibility complex. *Biomolecules*, 2014, vol. 4 (4), 994-1025
- **WILK, S.** ; **W.E. CHEN** ; **R.P. MAGNUSSON**. Properties of the nuclear proteasome activator PA28gamma (REGgamma. *Arch Biochem Biophys*, 2000, vol. 383 (2), 265-71
- **YEWDELL, J.W.** ; **L.C. ANTON** ; **J.R. BENNINK**. Defective ribosomal products (DRiPs): a major source of antigenic peptides for MHC class I molecules?. *J Immunol*, 1996, vol. 157 (5), 1823-6
- **WOLFERS, J.** ; **LOZIER, A.** ; **RAPOSO, G.** ; **REGNAULT, A.** ; **THERY, C.** ; **MASURIER, C.** ; **FLAMENT, C.** ; **POUZIEUX, S.** ; **FAURE, F.** ; **TURSZ, T.** Tumor-derived exosomes are a source of shared tumor rejection antigens for CTL cross-priming.. *Nat Med*, 2001, vol. 7, 297-303
- **ZITVOGEL, L.** ; **REGNAULT, A.** ; **LOZIER, A.** ; **WOLFERS, J.** ; **FLAMENT, C.** ; **TENZA, D.** ; **RICCIARDI-CASTAGNOLI, P.** ; **RAPOSO, G.** ; **AMIGORENA, S.** Eradication of established murine tumors using a novel cell-free vaccine: dendritic cell-derived exosomes.. *Nat Med*, 1998, vol. 4, 594-600